# EUROPEAN PATENT SPECIFICATION

(11) **EP 4 413 161 B1**
(45) Date of publication and mention of the grant of the patent: **15.04.2026**
(21) Application number: 22800619.3
(22) Date of filing: 04.10.2022
(51) Int. Cl.: C12Q 1/6869, C12Q 1/6874

(54) **METHOD OF DETERMINING A TARGET POLYMER IN A SAMPLE BY USING A GUIDE POLYMER**
VERFAHREN ZUR BESTIMMUNG EINES ZIELPOLYMERS IN EINER PROBE UNTER VERWENDUNG EINES LEITPOLYMERS
MÉTHODE DE DÉTERMINATION D'UN POLYMÈRE CIBLE DANS UN ÉCHANTILLON À L'AIDE D'UN POLYMÈRE DE GUIDAGE

(30) Priority: 04.10.2021 GB 202114183
(43) Date of publication of application: 14.08.2024
(73) Proprietor: Oxford Nanopore Technologies PLC, Oxford OX4 4DQ (GB); Monash University, Clayton, Victoria 3800 (AU)
(72) Inventor: JAYASINGHE, Lakmal Nishantha, Oxford OX4 4DQ (GB); WALLACE, Elizabeth Jayne, Oxford OX4 4DQ (GB); GUTIERREZ, Richard Alexander, Oxford OX4 4DQ (GB); DUNSTONE, Michelle Anne, Clayton, Victoria 3800 (AU); SPICER, Bradley Alan, Clayton, Victoria 3800 (AU)
(74) Representative: Greaves Brewster LLP
(86) International application number: PCT/EP2022/077537
(87) International publication number: WO 2023/057432

(56) References cited:
- WO-A1-2018/060740
- WO-A2-2016/028843
- WILES MICHAEL V ET AL: "CRISPR-Cas9-mediated genome editing and guide RNA design", MAMMALIAN GENOME, SPRINGER NEW YORK LLC, US, vol. 26, no. 9, 20 May 2015 (2015-05-20), pages 501 - 510, XP035600403, ISSN: 0938-8990, [retrieved on 20150520], DOI: 10.1007/S00335-015-9565-Z

## Description

### TECHNICAL FIELD

The invention relates generally to a method of detecting and/or analysing target polymers, especially target polynucleotides, using a biological pore. The invention also relates to a novel system for carrying out the method. The method has many uses. In particular, the method may be used for diagnosis, detection of polymorphisms and V(D)J repertoire analysis.

### BACKGROUND

There is currently a need for rapid and cheap polymer (e.g., DNA or RNA) sequencing and identification technologies across a wide range of applications. Existing technologies are slow and expensive mainly because they rely on amplification techniques to produce large volumes of polynucleotide and require a high quantity of specialist fluorescent chemicals for signal detection.

Biological pores (and other nanopores) have great potential as direct, electrical biosensors for polymers and a variety of small molecules. In particular, recent focus has been given to nanopores as a potential DNA sequencing technology.

When a potential is applied across a nanopore, there is a change in the current flow when an analyte, such as a nucleotide, resides transiently in the barrel for a certain period of time. Nanopore detection of the nucleotide gives a current change of known signature and duration. In the strand sequencing method, a single polynucleotide strand is passed through the pore and the identities of the nucleotides are derived. Strand sequencing can involve the use of a molecular brake to control the movement of the polynucleotide through the pore.

Solid-state pores have been used to identify target DNA sequences using Cas9-containing probes (Yang et al., Nano Lett. 2018, 18, 10, 6469-6474 and Weckman et al., ACS Sens. 2019, 4, 8, 2065-2072).

### SUMMARY OF THE INVENTION

The present invention is defined by the appended claims.

The present inventors have identified a novel use for biological pores in combination with guide polymers, such as RNAs and DNAs, and polymer-guided effector proteins, such as RNA-guided and DNA-guided effector proteins that form part of the CRISPR gene editing machinery. In particular, the present inventors have designed modified guide RNA sequences that can be used in conjunction with associated RNA-guided effector proteins to test for the presence, absence, or amount of one or more target polynucleotides in a sample. The present inventors have developed methods of detecting target polymers using guide polymers, such as RNAs, and polymer-guided effector proteins, such as guide RNA-guided effector proteins, in conjunction with a biological pore. In particular, the inventors have surprisingly identified that the target polymer and the polymer-guided effector protein interact differently with the biological pore and result in different electrical measurements. These differences between the electrical measurements may be used to identify the presence or absence of the target polymer more efficiently. The changes in electrical measurements resulting from the interaction between the polymer-guided effector protein and the biological pore are completely different from those observed with solid-state pores (Weckman et al., ACS Sens. 2019, 4, 8, 2065-2072 and Yang *et al. supra*). With a biological pore, the polymer-guided effector protein results in a longer, more pronounced, and more complex electrical effect than that associated with the target polymer. This type of effect is not seen with solid-state pores (Yang *et al.* and Weckman *et al. supra* saw a short, sharp blockade in the current resulting from Cas9). The inventors believe this surprising electrical effect results from an unexpected interaction with the biological pore. As discussed in more detail below, different polymer-guided effector proteins are capable of producing different electrical effects (*i.e.*, different signals) and this may be exploited in the context of the invention (*e*.*g*., to detect different target polymers).

The methods can be performed in a variety of ways, but have in common that they use guide polymers, such as guide RNAs, and polymer-guided effector proteins, such as RNA-guided effector proteins, to select the target polymer(s), such as target polynucleotide(s), and involve the delivery of a complex comprising the target polymer, guide polymer and polymer-guided effector protein to a biological pore. The methods can be extended to other polymer-guided protein effector systems, including the RNA editing system using C2c2. The guide polymer, such as guide RNA, may be specially adapted for use in a nanopore-based detection method.

The methods developed by the present inventors are sensitive and can be used to detect trace amounts of the target polymer in a sample without requiring a separate separation step to extract the target polymer(s) from other components in the sample. Thus, the methods are simple and do not require complex steps, such as enrichment or purification steps. Accordingly, the methods are rapid and can be used to obtain quick results and results from crude and/or "dirty" samples. The methods are therefore particularly useful in diagnostic settings where rapid diagnosis is required. The methods are also particularly useful in targeting a particular fragment or region of a gene or genome. A key benefit of the methods is that the polymer-guided effector protein does not actively need to be removed from the target polymer prior to measurement of the target or the adaptor. The target polymer is typically detected or characterized whilst still bound to the polymer-guided effector protein. In some embodiments, the method uses specifically designed guide polymers to facilitate separation of the target polymer(s) from other components in the sample.

The methods enable the regions of interest in a polymer sequence, such as a polynucleotide sequence, to be characterized, for example sequenced, in a sample that contains many other polymer sequences as it inherently includes a separation step. For example, only genes of interest present in a large genome may be sequenced. The sequencing can be limited to regions that contain SNPs, or to other regions of interest, such as V(D)J regions in T-cells. This improves sensitivity and efficiency, with the desired information being accessed without requiring complicated or time-consuming fragmentation or pull-down sample preparation methods. It also reduces the time taken perform the nanopore experiment as only fragments of the target polymer, such as target polynucleotide, of interest are measured, or an increased proportion thereof are measured relative to the total polymer fragments in the sample In cases where the amount of non-target polymer is in excess to that of the target polymer, the time taken to detect the target polymer can be significantly reduced due to the removal of or a reduction in the need to measure non-target polymer. The methods also benefit from not requiring PCR or other target enrichment approaches.

The invention provides:
- a method of determining the presence or absence of a target polymer in a sample comprising:
   a) contacting the sample with a guide polymer that (i) binds to a part of the target polymer and (ii) binds to or is attached to a polymer-guided effector protein, wherein the guide polymer and polymer-guided effector protein form a complex with any target polymer present in the sample;
   b) contacting the sample with a membrane comprising a biological pore with an opening that permits translocation of the complex through the pore;
   c) applying a potential difference across the membrane and taking one or more electrical measurements; and
   d) monitoring for the presence or absence of an electrical effect resulting from the translocation of the complex through the pore and thereby determining the presence or absence of the target polymer in the sample; and
- a system for determining the presence or absence of a target polymer in a sample comprising:
   a) a guide polymer that (i) binds to a part of the target polymer if present and (ii) binds to or is attached to a polymer-guided effector protein, wherein the guide polymer and polymer-guided effector protein form a complex with any target polymer present in the sample; and
   b) a biological pore with an opening that permits translocation of the complex through the pore.

### DESCRIPTION OF THE FIGURES

It is to be understood that Figures are for the purpose of illustrating particular embodiments of the invention only and are not intended to be limiting.

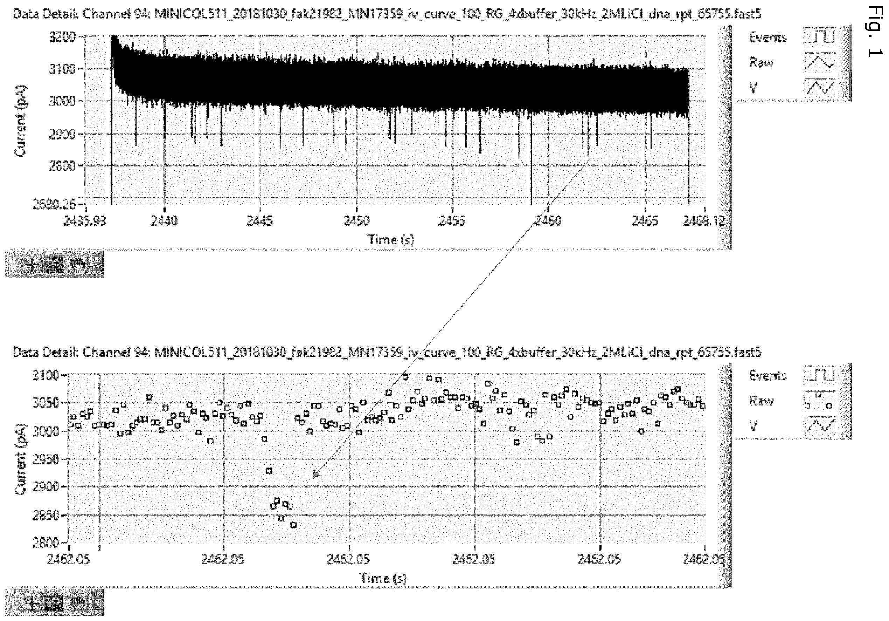

Fig. 1 shows an example of a current trace obtained at 100mV with 5nM 3.6kb DNA in the cis chamber. The spikes in the signal correspond to translocations of the DNA through the pore.

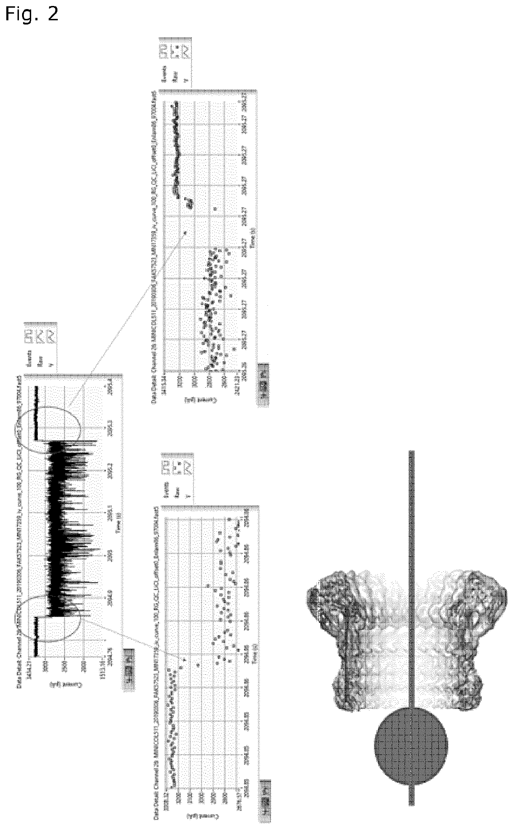

Fig. 2 shows an example of a current trace obtained at 80mV in which a DNA sample with a single Cas9 probe attached is in the flowcell. The Cas9 probe has Enlam86 as the crRNA which binds to position 3213 on the 3.6kb strand which is ~387 bases from one end. In this example the end of the DNA with the Cas9 closest to it enters the pore first as shown in the cartoon and evidenced by a signal attributed to DNA being observed after the Cas9 signal and not before.

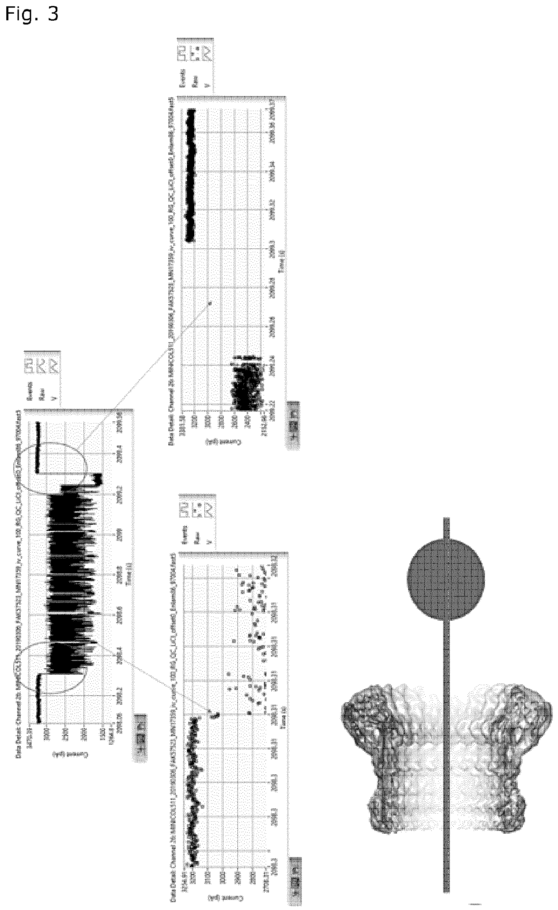

Fig. 3 shows an example of a current trace obtained at 80mV in which a DNA sample with a single Cas9 probe attached is in the flowcell. The Cas9 probe has Enlam86 as the crRNA which binds to position 3213 on the 3.6kb strand which is ~387 bases from one end. In this example the end of the DNA with the Cas9 closest to it enters the pore last as shown in the cartoon and evidenced by a signal attributed to DNA being observed before the Cas9 signal and not after.

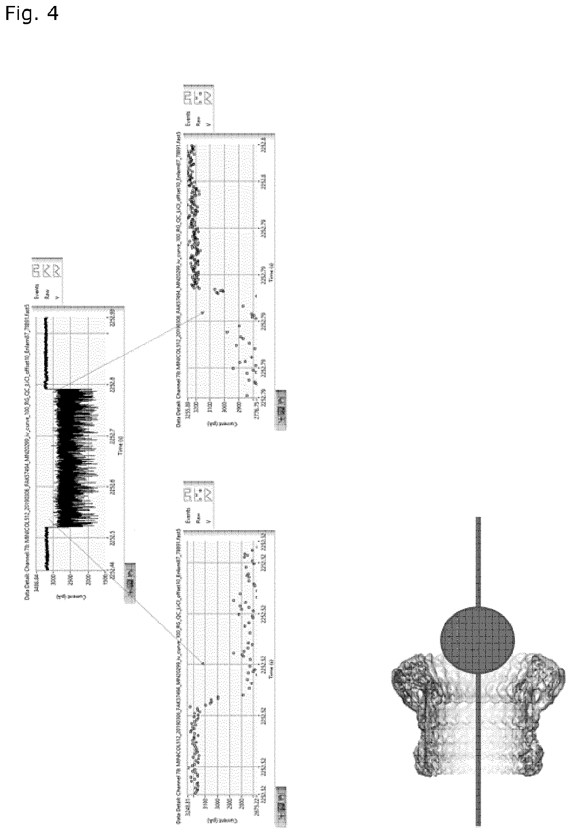

Fig. 4 shows an example of a current trace obtained at 80mV in which a DNA sample with a single Cas9 probe attached is in the flowcell. The Cas9 probe has Enlam87 as the crRNA which binds to position 2057 on the 3.6kb strand which is close to the middle of the DNA. Signal attributed to DNA is observed both before and after the Cas9 signal.

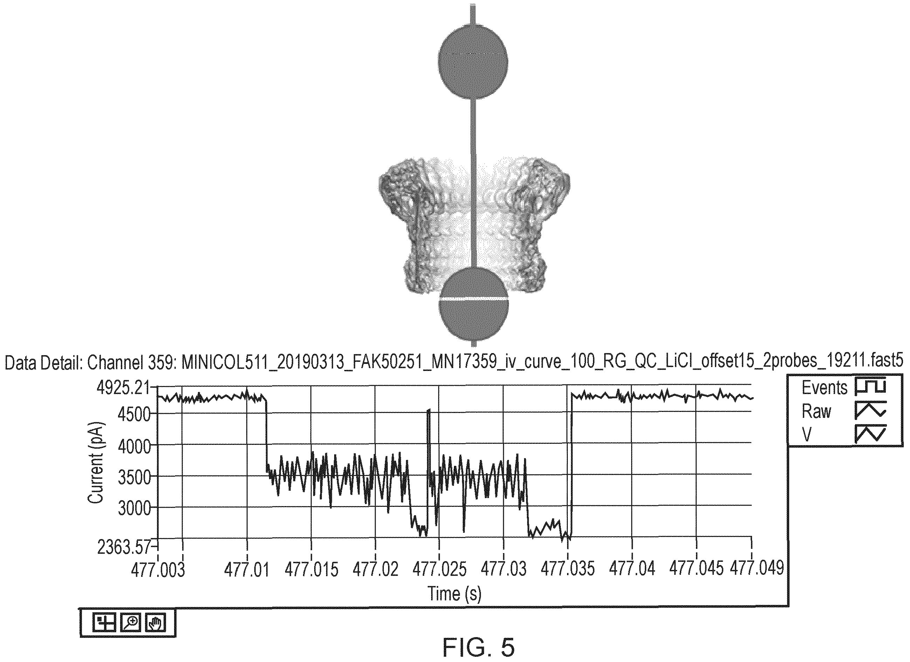

Fig 5 shows an example of a current trace obtained at 100mV in which a DNA sample with two Cas9 probes attached is in the flowcell. The Cas9 probes have either AR837 or Enlam51 which bind to positions 218 and 3368 respectively on the 3.6kb strand and result in a separation of over 3000 bases. Signal attributed to the DNA is observed between the two Cas9 signals but not before the first or after the second.

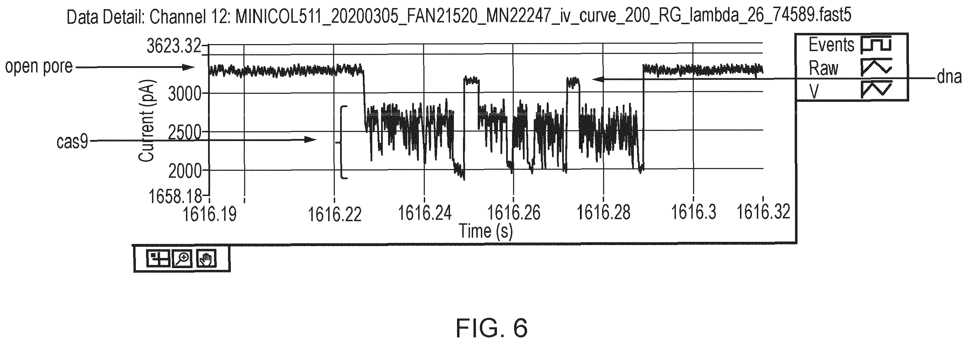

Fig. 6 shows an example of a current trace obtained at 800mV in which a DNA sample with three Cas9 probes attached is in the flowcell. The Cas9 probes have either Enlam5, Enlam26 or Enlam45 which bind to positions 4010, 25515 and 44609 respectively on the full-length lambda strand and result in a separation of approximately 20000 bases between probes. Signal attributed to the DNA is observed between the three Cas9 signals. This figure is annotated with what the different parts correspond to.

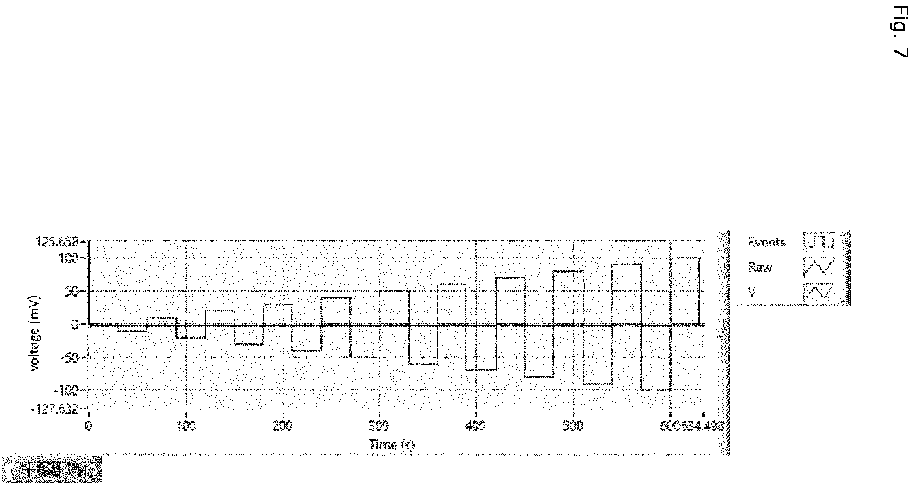

Fig.7 shows schematic of the IV curve voltage profile.

### DETAILED DESCRIPTION

It is to be understood that different applications of the disclosed products and methods may be tailored to the specific needs in the art. It is also to be understood that the terminology used herein is for the purpose of describing particular embodiments of the invention only, and is not intended to be limiting.

In addition, as used in this specification and the appended claims, the singular forms "a", "an", and "the" include plural referents unless the content clearly dictates otherwise. Thus, for example, reference to "a polymer" includes two or more polymers, "a polynucleotide" includes two or more polynucleotides, reference to "an anchor" refers to two or more anchors, reference to "a helicase" includes two or more helicases, and reference to "a pore" includes two or more pores and the like.

### Terminology

In all instances herein, the language "determining the presence or absence of" the target polymer is interchangeable "detecting" a target polymer. The method of the invention may therefore relate to a method of detecting a target polymer and step (d) may comprise detecting an electrical effect resulting from the translocation of the complex through the pore and thereby detecting the target polymer in the sample. The system of the invention may be for detecting a target polymer in a sample.

### Methods of the invention

The invention provides a method of determining the presence or absence of a target polymer in a sample comprising four steps. In step (a), the sample is contacted with a guide polymer that (i) binds to a part of the target polymer and (ii) binds to or is attached to a polymer-guided effector protein, wherein the guide polymer and polymer-guided effector protein form a complex with any target polymer present in the sample. The sample is contacted with the guide polymer and the polymer-guided effector protein. In step (b), the sample is contacted with a membrane comprising a biological pore with an opening that permits translocation of the complex through the pore. In step (c), a potential difference is applied across the membrane and one or more electrical measurements are taken. In step (d) the presence or absence of an electrical effect resulting from the translocation of the complex through the pore is monitored and the presence or absence of the target polymer in the sample is determined. The complex comprises the target polymer, if present, and the polymer-guided effector protein and so the electrical effect resulting from the translocation of the complex through the pore is capable of providing information about both the target polymer and the protein. This is discussed in more detail below. Step (a) and (b) may be carried out simultaneously or sequentially in either order.

The method may comprise (a) contacting the sample with a guide polymer that (i) binds to a sequence in the target polymer and (ii) binds to or is attached to a polymer-guided effector protein to form a complex, wherein the sample is in contact with a membrane comprising a biological pore with a constriction that permits translocation of the complex through the pore and wherein a potential is applied to the pore; and (b) taking one or more electrical measurements as at least a portion of the complex moves with respect to the pore to detect the presence or absence of the complex, thereby determining the presence or absence of the target polymer in the sample. Step (b) may comprise measuring the current flowing through the pore.

In some embodiments, the method comprises: (a) contacting the sample with a guide polymer that binds to a sequence in the target polymer, a polymer-guided effector protein and a membrane comprising a biological pore with a constriction that permits translocation of the complex through the pore; (b) applying a potential difference across the membrane; and (c) taking one or more electrical measurements, or measuring another signal resulting from the interaction of the sample, guide polymers and polymer-guided effector proteins with the pore, to determine the presence or absence of a complex comprising the guide polymer, polymer-guided effector protein and the target polymer, thereby determining the presence or absence of the target polymer in the sample. Step (c) may comprise measurement of the current flowing through the pore.

The methods can be carried out in a number of different ways. The methods can be used to perform a variety of different applications. The methods may be used, for example, to determine the presence or absence of a single target polymer, or of a number of target polymers. The methods may be quantitative. For example, the amount (such as the concentration) of the target polymer present in a sample may be determined using a method of the invention and/or relative amounts of different polymers present in a sample may be determined. The methods can provide further information about a target polymer, such as the presence or absence of a polymorphism and/or the identity of a polymorphism.

The method may comprise determining whether an adaptor attached to the guide polymer interacts with the pore. The method may be carried out such that guide polymers comprising adaptors that are not bound to the target polymer do not interact with the pore. Typically, such unbound guide polymers are washed away before a transmembrane potential is applied to the membrane. The target polymer may be tethered to a surface, for example to the membrane, to prevent bound guide polymers comprising adaptors from being washed away. The target polymer may have a tether, such as a membrane anchor, attached to it directly, for example, to one of its ends. Alternatively, a second guide polymer/polymer-guided effector protein which comprises a tether, such as a membrane anchor, may be used to tether the target polymer to the surface, for example to the membrane.

The surface to which the target polymer is tethered may be a bead.

The adaptor is typically unique to the target polymer and produces a distinct signal on interacting with a pore. Multiple guide polymers, each selective for a different target polymer and having a different adaptor may be added to the sample to detect and/or quantify different target polymers on the basis of the different signals caused by the different adaptors interacting with the pore. In this embodiment, the adaptors may be considered to comprise barcodes.

The method may use multiple guide polymers that bind to different polymer sequences. The different polymer sequences may, for example, be different sequences in the same target polymer (*e*.*g*., different portions of the target polymer), sequences of different target polymers or alternative sequences within a target polymer, such as sequences that encompass polymorphisms, for example single nucleotide polymorphisms (SNPs). Step (a) preferably comprises contacting the sample with two or more guide polymers, wherein the two or more guide polymers bind to different sequences in the target polynucleotide or to sequences of different target polynucleotides.

In some embodiments, the method may comprise further characterizing the target polymer. For example, the method may comprise sequencing all or part of the target polymer, such as target polynucleotide.

In some embodiments, the method uses two or more guide polymers and two or more polymer-guided effector proteins. For instance, step (a) preferably comprises contacting the sample and the pore with two or more guide polymers each of which bind to a part of the target polymer and two or more polymer-guided effector proteins, wherein the two or more guide polymers and two or more polymer-guided effector proteins form two or more complexes with any target polymer present in the sample. Any number of two or more guide polymers and guide polymer-guided effector proteins may be used, such as 2, 3, 4, 5, 6, 7, 8, 9, 10 or more. The number of the two or more guide polymers is typically the same as the number of the two or more guide polymer-guided effector proteins. The number of complexes is typically the same as the number of the two or more guide polymers and the number of two or more guide polymer-guided effector proteins and may be any of the numbers listed above. In these embodiments, step (d) preferably comprises monitoring for the presence or absence of electrical or current effects resulting from the translocation of the two or more complexes through the pore and thereby determining the presence or absence of the target polymer in the sample. The number of electrical or current effects is typically the same as the number of two or more complexes. The two or more guide polymers preferably bind to different sequences or parts of the target polymer. Those sequences or parts may be present in different target polymers, within the same target polymer or may be alternative sequences, such as SNPs, that may be present in the target polymer. The two or more guide polymers preferably bind to different parts of the same target polymer. This means two or more, such as such as 2, 3, 4, 5, 6, 7, 8, 9, 10 or more, complexes form on an individual target polymer, translocate through the pore, and result in multiple electrical or current effects. This is discussed in more detail below. The two or more polymer-guided effector proteins are preferably different polymer-guided effector proteins or different RNA-guided effector proteins. The two or more polymer-guided effector proteins are preferably different polymer-guided effector proteins or different RNA-guided effector proteins preferably result in different electrical effects. The two or more polymer-guided effector proteins may be any of those discussed in more detail below. Two proteins are different if they are different proteins or protein homologs, such as Cas and Cpf1, or if they are derived from the same protein, such as Cas9, and one of them is modified, for instance via substitution, deletion, or addition, or both of them are modified in different ways.

In some embodiments, the method may comprise detecting the presence, absence or amount of a target polymer using two or more guide polymers and/or two or more polymer-guided effector proteins which bind to different regions of the target polymer and form two or more complexes, wherein binding of two or more different guide polymer/polymer-guided effector proteins to the target polymer results in a detectable electrical signal, for example a detectable current change, through a biological pore that permits translocation of the complexes through the pore if the target polymer is present in the sample. The signal may be characteristic of an adaptor in one of the guide polymers/ polymer-guided effector complexes, with the signal only or primarily being observed when that guide polymer is "linked" to a second guide polymer/polymer-guided effector protein comprising a membrane anchor, and this "linkage" occurs when both guide polymers/polymer-guided effector proteins are bound to the target polymer. In other words, the target polymer serves to "link" the guide polymers/ polymer-guided effector complex comprising an adaptor to the guide polymers/ polymer-guided effector complex comprising a membrane anchor. In the embodiment wherein the polymer-guided effector protein comprises a membrane anchor, attachment may be via the protein itself, for example by use of a strep-tag/flag-tag/his-tag.

In some embodiments, the method may comprise selectively characterizing, for example sequencing, target polymers using a biological pore that permits translocation of the complex through the pore by marking each target polymer with a guide polymer/polymer-guided effector protein complex specific for the target polymer such that the target polymer can be selectively sequenced without needing to separate the target polymer from other polymers in the sample prior to contacting the sample with the pore.

For example, the guide polymer/polymer-guided effector protein complex may be tagged with a membrane anchor so that only target polymers are tethered to the membrane. Other polymers in the sample may be washed away. Alternatively, a polymer binding protein capable of moving along a polymer may be bound to the end of the polymers in the sample, for example using techniques known in the art, and the polymer binding protein may be caused to move along the polymers after complex formation, for example by adding a cofactor necessary for movement of the polymer binding protein. In this embodiment, the bound guide polymer/ polymer-guided effector protein complex stalls the polymer binding protein on the target polymer, whilst the polymer binding protein is processed off the ends of non-target polymers. Then, when the transmembrane potential is applied, the force of the potential and the contact with the pore displaces the bound guide polymer/polymer-guided effector protein complex from the target polymers so that the target polymer translocates through the pore. The non-target polymers to which no polymer binding protein is bound pass through the pore so rapidly that no signal is detected, or so that any signal obtained can easily be discriminated from signals resulting from the interaction of the target polymer with the pore. The 3'-terminated strands of the polymers in the sample, including both target and non-target polymers, may be degraded, for example using an exonuclease. In this way the target polymer can be selectively characterized, for example sequenced. The polymer binding protein may be caused to move along the polymers by adding a cofactor, such as ATP or another nucleoside for example and γsGTP.

In another embodiment, the polymer-guided effector protein can be used to cut the polymer at a selected point. This may be used to limit the information, such as sequence information, obtained by the method about a region of interest. For example, two polymer-guided effector proteins with nuclease activity may be used to obtain a polymer fragment of interest. As an alternative, a modified polymer-guided effector protein having inactivated or disabled nuclease activity may be used to stall a polymer binding protein as described above and a second polymer-guided effector protein may be used to truncate the fragment being characterized. This embodiment is, for example, particularly useful in V(D)J repertoire analysis applications.

Further, in some embodiments, the guide polymer/polymer-guided effector protein complex tags or labels the target polymer such that the effect of the guide polymer/polymer-guided effector protein complex on the current passing through the pore can be used to determine the presence or absence of, quantify or identify the target polymer.

For example, the guide polymer or polymer-guided effector protein may be attached to an adaptor that may be used to identify a target polymer tagged with a membrane anchor because the adaptor will only interact with the pore when it is bound to the target polymer tethered to the membrane. Unbound guide polymers and polymer-guided effector proteins may be washed away. Multiple guide polymers, each selective for a different target polymer and having a different adaptor may be added to the sample to detect and/or quantify different target polymers on the basis of the different signals caused by the different adaptors interacting with the pore.

Alternatively, the guide polymer/polymer-guided effector protein complex bound to the target polymer may produce a detectable signal when the target polymer passes through a biological pore that permits translocation of the complex through the pore.

Since the biological pore is sufficiently large to allow the passage of polymers and bound guide polymer/polymer-guided effector protein complex, the passage of the polymer/guide polymer/polymer-guided effector protein complex through the pore will produce a recognisable signal when the guide polymer/polymer-guided effector protein complex passes through the pore. This signal is discussed in more detail below. Thus, one or more guide polymer/polymer-guided effector protein complexes may be used to identify a target polymer. For example, a guide polymer may be designed to bind only if a particular polymorphism is present in the target polymer. The number of signals attributable to bound guide polymer/polymer-guided effector protein complexes observed as the target polymer passes through the pore, or the presence or absence of a particular signal as the target polymer passes through the pore may indicate the presence or absence of the polymorphism. One or more of the guide polymer, polymer-guided effector protein and/or target polymer are typically modified to enable the method to be carried out.

The method may further comprise determining the amount of the target polymer, such as the target polynucleotide, or one or more characteristics of the target polymer, such as the target polynucleotide. The one or more characteristics are typically selected from (i) the length of the target polymer or polynucleotide, (ii) the identity of the target polymer or polynucleotide, (iii) the sequence of the target polymer or polynucleotide, (iv)the secondary structure of the target polymer or polynucleotide and (v) whether or not the target polymer or polynucleotide is modified. These features are described in WO 2018/060740. Any combination of (i) to (v) may be measured in accordance with the invention, such as {i}, {ii}, {iii}, {iv}, {v}, {i,ii}, {i,iii}, {i,iv}, {i,v}, {ii,iii}, {ii,iv}, {ii,v}, {iii,iv}, {iii,v}, {iv,v}, {i,ii,iii}, {i,ii,iv}, {i,ii,v}, {i,iii,iv}, {i,iii,v}, {i,iv,v}, {ii,iii,iv}, {ii,iii,v}, {ii,iv,v}, {iii,iv,v}, {i,ii,iii,iv}, {i,ii,iii,v}, {i,ii,iv,v}, {i,iii,iv,v}, {ii,iii,iv,v} or {i,ii,iii,iv,v}. In all of (i)-(v), the target polymer is preferably a target polynucleotide.

Step (a) may further comprise contacting the sample with beads (*e*.*g*., microparticles) to which one or more components of the complex can bind. Alternatively, one or more of the components used in (a), *e*.*g*., the target polymer, the guide polymer or the polymer-guided effector protein may be prebound to beads (*e*.*g*., microparticles). The guide polymer and/or polymer-guided effector protein preferably has a binding moiety capable of coupling to a bead attached thereto and in step (a) the guide polymer or polymer-guided effector protein is coupled to a bead, or step (a) further comprises contacting the sample with beads.

The sample may be provided in an aqueous medium or alternatively the sample may be added to an aqueous medium containing the polymer-guided effector protein and the guide polymer. The aqueous medium will typically comprise ions to provide ion flow through the pore upon application of a potential difference across the membrane. The aqueous medium will also typically comprise a buffer. The aqueous medium typically has a pH in the range of 6 to 9 and/or an ion concentration in the range of from about 100 to about 200 mM salt, such as NaCl.

The bead is typically denser than the aqueous medium and sink through the medium to contact the membrane, thus effectively enhancing the concentration of the species attached to the anchor at the membrane surface.

In the method, the applied potential may be a voltage potential. Alternatively, the applied potential may be a chemical potential. An example of this is using a salt gradient across an amphiphilic layer. A salt gradient is disclosed, for example, in Holden et al., J Am Chem Soc. 2007 Jul 11:129(27):8650-5.

The guide polymer and/or effector protein may be cross-linked to the target polymer.

The method may be used to detect one or more, such as 2, 3, 4, 5, 6, 7 8, 9, 10, 20, 30 or more, target polymers in a complex background following enrichment of the target molecule. In one embodiment, sequencing, *e*.*g*., nanopore sequencing, is used for the detection. Hence in this embodiment, the target DNA molecule may be identified primarily by its sequence.

The method may be carried out as a multiplex assay. The multiplex assay may utilize different barcodes. For instance, the different barcodes may be on different adaptors. The barcodes may, for example, each have a distinct nucleotide sequence enabling the barcodes to be identified by the pore. In one embodiment a barcode sequence may be ligated to all polymers in a sample, prior to contacting the sample with the guide polymer and polymer-guided binding protein. A second barcode can be added to a second sample, prior to contacting the sample with the guide polymer and polymer-guided binding protein. The first and second samples can be combined prior to or after addition of the guide polymer and polymer-guided binding protein, preferably after the guide polymer and polymer-guided binding protein have bound to the target polymers. Where pooling of the samples occurs after the guide polymer and polymer-guided binding protein have bound to the target polymers, purification steps (including stress, removal of non-target bound protein, and/or removal of non-target polymers) may be carried out prior to or after pooling. Multiple samples, such as 2, 3, 4, 5, 6, 7 8, 9, 10, 20, 30 samples, can be labelled with barcodes and then combined in this way. In this embodiment all the samples can be sequenced simultaneously, e.g., using the same flowcell, and identified using their barcode adapter.

In another embodiment, barcodes and sequencing adapters may be added after the guide polymer and polymer-guided binding protein have bound to the target polymers. For example, the barcodes and sequencing adaptors may be ligated on beads. The target-loaded, barcoded, adapted beads may be added to the sample after the guide polymer and polymer-guided binding protein have bound to the target polymers and optionally after one or more purification staps (such as stress, removal of non-target bound protein, and/or removal of non-target polymers) have been carried out.

The method may comprise removing any polymer-guided effector protein and/or guide polymer, *e*.*g*., guide polymer/polymer guided effector protein complex, that is not specifically bound to the target polymer. The excess polymer-guided effector protein and/or guide polymer, *e*.*g*., guide polymer/polymer guided effector protein complex, present in the sample, which is not bound to target polymer can produce background when monitoring the interaction of the target polymer/guide polymer/polymer guided effector protein complex with the pore. Guide polymer, polymer-guided effector protein and/or polymer-guided effector protein /guide polymer complex that is not specifically bound to the target polymer may be separated from the complex comprising the guide polymer, polymer-guided effector protein and target polymer by binding the polymers in the sample a surface, *e*.*g*., beads or a column. The target polymers may also be separated from non-target polymers in the sample by binding the guide polymer, polymer-guided effector protein and/or polymer-guided effector protein /guide polymer complex in the sample to a surface, *e*.*g*., beads or a column. The target polymer(s) may, for example, be separated from the background by means of a 'pulldown' via a capture moiety on the guide polymer/polymer-guided effector protein complex.

The method may comprise selectively denaturing any polymer-guided effector protein that is not specifically bound to the target polymer prior to step (b). 'Off-target' effects of guide polymer/polymer-guided effector protein complex binding may be reduced by applying a thermal and/or chemical stress to the bound guide polymer/polymer-guided effector protein complex. Typically, in this embodiment, non-target bound polymer-guided effector protein is selectively denatured by the heat stress or chemical stress applied. The applied heat or chemical treatment can be selected such that only free polymer-guided effector protein (*i*.*e*., polymer-guided effector protein that is not bound to polymers in the sample, but which may be bound to guide polymer) and non-target bound polymer-guided effector protein (*i.e.*, polymer-guided effector protein that is bound non-specifically to polymers in the sample, or "off target" polymer-guided effector protein) is denatured. Target-bound polymer-guided effector protein remains bound to the target polymer during the heat stress or chemical stress. Any off-target polymer-guided effector protein is released from its non-specific binding to the polymers in the sample. In one embodiment, only polymer-guided effector protein bound to a target sequence that is exactly complementary to the corresponding sequence in the guide polymer remains bound to a polymer during the stress.

Any suitable chemical stress can be used, such as urea (*e*.*g*., up to 6M, 5M or 4M), guanidinium hydrochloride, extreme pH (acidic or alkaline, such as below pH6, pH5 or pH4 or above pH8, pH9 or pH10) or high salt concentrations. Suitable conditions may readily be determined by the skilled person.

The chemical stress may be carried out for any time period that results in the selective disruption of non-specific binding of polymer-guided effector protein to polymers, without disrupting specific binding of polymer-guided effector protein to target polymer. The chemical stress may be carried out for from about 30 seconds to about 10 minutes, such as for about 1 minute, about 2 minutes, about 3 minutes, about 5 minutes, about 6 minutes, about 7 minutes, about 8 minutes or about 9 minutes.

The heat stress may be carried out at any suitable temperature. Typically, the temperature is high enough to disrupt non-specific binding of polymer-guided effector protein to polymers but is low enough that specific binding of polymer-guided effector protein to target polymer is not disrupted. For example, the sample may be heated to a temperature of from about 40^{O}C to about 65^{O}C, such as from about 45°C to about 65°C, from about 50°C to about 60^{O}C, or about 55^{O}C.

The heat stress may be carried out for any time period that results in the selective disruption of non-specific binding of polymer-guided effector protein to polymers, without disrupting specific binding of polymer-guided effector protein to target polymer. The heat stress may be carried out for from about 30 seconds to about 10 minutes, such as for about 1 minute, about 2 minutes, about 3 minutes, about 5 minutes, about 6 minutes, about 7 minutes, about 8 minutes or about 9 minutes.

A purification step may be used to remove excess, unbound polymer-guided effector protein and/or guide polymer. This may be achieved, for example, by adding polyethylene glycol (PEG) and sodium chloride to the sample and contacting the sample with paramagnetic beads coated with carboxyl groups such that the polymers present in the sample bind to the beads. Suitable beads include commercially available SPRI beads and standard protocols known in the art may be used. In one embodiment, the target polymer/guide polymer/polymer-guided effector protein complex may subsequently be separated from non-target polymers using a surface, *e*.*g*., a different capture bead. Typically, here the guide polymer/polymer-guided effector protein may contain a binding moiety that is used to specifically bind the target polymer/guide polymer/polymer-guided effector protein complex to the surface. Any non-bound polymers may be washed away. The target polymer/guide polymer/polymer-guided effector protein complex may be eluted from the surface by any suitable means, or the surface may be used to deliver the complex to a pore, *e*.*g*., where the surface is beads.

'Off-target' effects may be minimised further by purification the target polymer/guide polymer/polymer-guided effector protein complexes on a capture surface using a first binding moiety on the guide polymer/polymer-guided effector protein, *e*.*g*., on the guide polymer, eluting the target polymer/guide polymer/polymer-guided effector protein complexes and transferring the target polymer/guide polymer/polymer-guided effector protein complexes to a second specific capture surface using a second binding moiety on the guide polymer/polymer-guided effector protein, *e*.*g*., on the guide polymer. This can be achieved, for example, where the first binding moiety and the second binding moiety are both end extensions, or other single stranded polymer sequences capable of binding to an oligonucleotide, on the guide polymer. The first end extension on the guide polymer has a sequence complementary to a first capture oligonucleotide on a first capture surface, *e*.*g*., a bead, and the second end extension on the guide polymer has a sequence complementary to a second capture oligonucleotide on a second capture surface, *e*.*g*., a bead. One way of configuring this is the crRNA comprising a 3' DNA extension used for capture of the target polymer on a bead, column or surface and the tracrRNA comprises a 5' DNA extension. The release of the target from the first capture surface, *e*.*g*., a bead, may be affected by the phenomenon known as toehold displacement.

The target polymer/guide polymer/polymer-guided effector protein complex is first separated from non-target polymers by capture on beads bearing a first capture oligonucleotide complementary to the first end extension. Non-target polymers are washed away. The target polymer/guide polymer/polymer-guided effector protein complex may be eluted from the bead by toehold displacement, via the addition of a competitor oligonucleotide that competes for the binding to the bead with the first end extension on the guide polymer. In this embodiment, the first capture oligonucleotide is longer than the first end extension and comprises a first sequence and a second sequence, wherein the first sequence is complementary to a sequence in the first end extension and the first and second sequences are both complementary to the sequence of the competitor oligonucleotide. Typically, the first sequence has a length of from about 5 to about 40, such as from about 10 to about 30 or from about 15 to about 25 nucleotides, for example about 20 nucleotides and the second sequence has a length of from about 5 to about 40, such as from about 10 to about 30 or from about 15 to about 25 nucleotides, for example about 20 nucleotides. The competitor oligonucleotide may have a length of from about 10 to about 80, such as from about 20 to about 60 or from about 30 to about 50 nucleotides, for example about 40 nucleotides. The first capture oligonucleotide may have a length of from about 10 to about 80, such as from about 20 to about 60 or from 30 to about 50 nucleotides, for example 40 nucleotides. The capture oligonucleotide may have the same length as the competitor oligonucleotide, or the capture oligonucleotide may be longer or shorter than the competitor oligonucleotide, provided that capture oligonucleotide and competitor oligonucleotide have sequences that are complementary over both the first and second sequences. In this embodiment, the first end extension comprises an end portion, which is at the 5' end in a 5' end extension or at the 3' end in a 3' end extension, which has a sequence that is not complementary to a sequence in the first capture oligonucleotide, and a portion that has a sequence that is complementary to the first sequence in the first capture oligonucleotide. The end portion of the first end extension may typically have a length of from about 2 to about 10 nucleotides, such as about 3, 4, 5 or 6 nucleotides. The portion of the first end extension that is complementary to the first capture oligonucleotide may typically have a length of from about 5 to about 40, such as from about 10 to about 30 or from about 15 to about 25 nucleotides, for example about 20 nucleotides.

Following elution of the target polymer/guide polymer/polymer-guided effector protein complex, the complex is bound to a second 'delivery' bead via second end extension on the guide polymer. The second 'delivery' bead comprises a second capture oligonucleotide that is complementary to the second end extension. The second capture oligonucleotide may have a length of from about 5 to about 40, such as from about 10 to about 30 or from about 15 to about 25 nucleotides, for example about 20 nucleotides or a length of from about 10 to about 80, such as from about 20 to about 60 or from about 30 to about 50 nucleotides, for example about 40 nucleotides. The second end extension may have a length of from about 5 to about 40, such as from about 10 to about 30 or from about 15 to about 25 nucleotides, for example about 20 nucleotides. The second capture oligonucleotide may have the same length as the second end extension, or the second capture oligonucleotide may be longer or shorter than the end extension, provided that second capture oligonucleotide and second end extension have sequences that are complementary over a length of from about 5 to about 40, such as about 10 to about 30 or from about 15 to about 25 nucleotides, for example about 20 nucleotides. The second end extension has a sequence that does hybridise to the first capture nucleotide, the first end extension or the competitor oligonucleotide. The second capture oligonucleotide also has a sequence that does hybridise to the first capture nucleotide, the first end extension or the competitor oligonucleotide.

Accordingly, where the guide polymer comprises a first end extension and a second end extension, and the method may comprise prior to step (b):
(i) contacting the sample with a surface having bound thereto a first capture oligonucleotide comprising a sequence complementary to the first end extension, such that the guide polymer/polymer-guided effector protein/target polymer complex is bound to the surface;
(ii) contacting the guide polymer/polymer-guided effector protein/target polymer complex bound to the surface with a competitor oligonucleotide, such that the guide polymer/polymer-guided effector protein/target polymer complex is released from the surface;
(iii) contacting the guide polymer/polymer-guided effector protein/target polymer complex with beads having bound thereto a second capture oligonucleotide comprising a sequence complementary to the second end extension, such that the guide polymer/polymer-guided effector protein/target polymer complex is bound to the beads; and optionally
(iv) delivering the beads to the pore.

The method preferably further comprises a step of washing away guide polymers, polymer-guided effector proteins and/or polynucleotides that are not coupled to the membrane and/or beads.

In different embodiments of the invention, there may be: (i) no heat or chemical stress, purification to remove excess, unbound and/or non-target bound polymer-guided effector protein or toehold purification; (ii) only heat or chemical stress; (iii) only purification to remove excess, unbound and/or non-target-bound polymer-guided effector protein; (iv) only toehold purification; (v) heat or chemical stress and purification to remove excess, unbound and/or non-target-bound polymer-guided effector protein; (vi) heat or chemical stress and toehold purification; (vii) purification to remove excess, unbound and/or non-target-bound polymer-guided effector protein and toehold purification; or (viii) heat or chemical stress, purification to remove excess, unbound and/or non-target-bound polymer-guided effector protein and toehold purification.

The beads to which the target polymer/guide polymer/polymer-guided effector protein complex is bound may be used to deliver the complex to the pore. For example, the beads may be magnetic and the target polymer bound to the beads may be drawn into the wells of a flowcell comprising the pore by the application of a magnetic field placed underneath the flowcell, or can be allowed to settle by gravity. Sequencing can be initiated by flowing tether, such as an oligonucleotide-cholesterol tether which hybridizes to the adaptor ends, over the beads, which tethers the beads to the membrane. Alternatively, the tether can be introduced into the membrane before the bead-target polymer conjugate is added. For example, an oligonucleotide-cholesterol tether which hybridizes to the adaptor ends may be integrated into the membrane in the flowcell by flowing running buffer and the tether through the flowcell before the beads-target polymer(s) are added. In this situation, when the beads-target polymer(s) are added to the flowcell, they become tethered to the membrane when they encounter the oligonucleotide that is anchored in the membrane by the cholesterol.

In some embodiments, the target polymers may be adapted for nanopore sequencing. For example, all of the polymers in the sample may have sequencing adaptors added to one or both ends prior to step (a) of the method. The polymers in the sample may be fragmented prior to addition of the sequencing adaptors. Alternatively, the target polymers may have sequencing adaptors added to one or both ends after step (a). After step (a) the polymers in the sample are preferably fragmented and/or the guide polymers and/or polymer-guided effector protein are preferably cross-linked to the target polymer. In this embodiment, the sequencing adaptors may be added before or after separation of the target from non-target polymers. The sequencing adaptor typically comprises a polymer binding protein that is capable of moving along the polymer. When the sequencing adaptor is added after step (a) the polymer-guided effector protein/guide polymer complex remains bound to the target polymer. After binding of the adaptor, in some embodiments, generally where the target polymer has been separated from non-target polymers prior to adaptor addition, the polymer-guided effector protein/guide polymer complex may be displaced by the polymer binding protein that is capable of moving along the polymer loaded on the adaptor. Displacement of the polymer-guided effector protein/guide polymer complex by the polymer binding protein that is capable of moving along the polymer can be controlled by the addition of one or more cofactor needed for the polymer binding protein to moving along a polymer.

The target polymer may be adapted for nanopore sequencing by ligation of adaptors to either or both of its free ends whilst bound to the surface, e.g., column or beads, via the guide polymer/polymer-guided effector protein. The ends may be dA-tailed to facilitate adaptor binding.

### Target polymer

The target polymer may be any polymer. Suitable polymers include, but are not limited to, oligonucleotides, polynucleotides, such as RNA or DNA, proteins, polypeptides, oligosaccharides, polysaccharides, or other polymers for which information about their presence or characteristics is of value.

The target polymer is preferably a target polynucleotide. The polynucleotide can be a nucleic acid, such as deoxyribonucleic acid (DNA) or ribonucleic acid (RNA). The polynucleotide can comprise one strand of RNA hybridised to one strand of DNA. The polynucleotide may be any synthetic nucleic acid known in the art, such as peptide nucleic acid (PNA), glycerol nucleic acid (GNA), threose nucleic acid (TNA), locked nucleic acid (LNA) or other synthetic polymers with nucleotide side chains. The PNA backbone is composed of repeating N-(2-aminoethyl)-glycine units linked by peptide bonds. The GNA backbone is composed of repeating glycol units linked by phosphodiester bonds. The TNA backbone is composed of repeating threose sugars linked together by phosphodiester bonds. LNA is formed from ribonucleotides as discussed above having an extra bridge connecting the 2' oxygen and 4' carbon in the ribose moiety.

The polynucleotide is preferably DNA, RNA or a DNA or RNA hybrid, most preferably DNA. The target polynucleotide comprises a double stranded region to which the guide-polynucleotide and polynucleotide-guided effector protein bind. The target polypeptide may be double stranded. The target polypeptide may comprise single stranded regions and regions with other structures, such as hairpin loops, triplexes and/or quadruplexes. The DNA/RNA hybrid may comprise DNA and RNA on the same strand. Preferably, the DNA/RNA hybrid comprises one DNA strand hybridized to an RNA strand.

The target polynucleotide can be any length. For example, the polynucleotides can be at least 10, at least 50, at least 100, at least 150, at least 200, at least 250, at least 300, at least 400 or at least 500 nucleotides or nucleotide pairs in length. The target polynucleotide can be 1000 or more nucleotides or nucleotide pairs, 5000 or more nucleotides or nucleotide pairs in length or 100000 or more nucleotides or nucleotide pairs in length. The target polynucleotide may be an oligonucleotide. Oligonucleotides are short nucleotide polymers which typically have 50 or fewer nucleotides, such 40 or fewer, 30 or fewer, 20 or fewer, 10 or fewer or 5 or fewer nucleotides. The target oligonucleotide is preferably from about 15 to about 30 nucleotides in length, such as from about 20 to about 25 nucleotides in length. For example, the oligonucleotide can be about 15, about 16, about 17, about 18, about 19, about 20, about 21, about 22, about 23, about 24, about 25, about 26, about 27, about 28, about 29 or about 30 nucleotides in length.

The target polymer may be a polymer associated with a disease and/or a microorganism.

The method may detect multiple, such as from about 2 to 50, 3 to 40, 4 to 30, 5 to 25, 6 to 15 or 8 to 10, target polymers. The target polymers may be a group of polymers. For instance, the group may be associated with a particular phenotype. The group may be associated with a particular type of cell. For instance, the group may be indicative of a bacterial cell. The group may be indicative of a virus, a fungus, a bacterium, a mycobacterium or a parasite.

The target polymers may be a group of two or more polymers that are, or comprise, biomarkers associated with a particular disease or condition. The biomarkers can be used to diagnose or prognose the disease or condition. Suitable panels of biomarkers are known in the art, for example as described in Edwards, A.V.G. et al. (2008) Mol. Cell. Proteomics 7, p1824-1837; Jacquet, S. et al. (2009), Mol. Cell. Proteomics 8, p2687-2699; Anderson N.L. et al (2010) Clin. Chem. 56, 177-185. The disease or condition is preferably cancer, heart disease, including coronary heart disease and cardiovascular disease, or an infectious disease, such as tuberculosis or sepsis.

The target oligonucleotide or polynucleotide is preferably a microRNA (or miRNA) or a small interfereing RNA (siRNA). The group of two or more target polynucleotides may be a group of two or more miRNAs. Suitable miRNAs for use in the invention are well known in the art. For instance, suitable miRNAs are stored on publically available databases (Jiang Q., Wang Y., Hao Y., Juan L., Teng M., Zhang X., Li M., Wang G., Liu Y., (2009) miR2Disease: a manually curated database for microRNA deregulation in human disease. Nucleic Acids Res.).

The target polymer preferably comprises a leader sequence. Such sequences are discussed in WO 2018/060740.

### Polymer-guided effector protein

The polymer-guided effector protein may be any protein that binds to the target polymer via a guide polymer. The polymer-guided effector protein may, by way of non-limiting examples, bind to or be attached to a guide oligonucleotide, such as an aptamer, or a guide polypeptide, such as an antibody, which binds to part of the target polymer.

The polymer-guided effector protein is preferably a polynucleotide-guided effector protein. In this embodiment, the guide polymer is preferably a guide polynucleotide. The polynucleotide-guided effector protein may be any protein that binds to or is attached to a guide polynucleotide and which binds to the target polymer, preferably a target polynucleotide, to which the guide polynucleotide binds. The polynucleotide-guided effector protein preferably comprises a target polynucleotide recognition domain and at least one nuclease domain. The recognition domain binds a guide polynucleotide (*e*.*g*., RNA) and a target polynucleotide (*e*.*g*., DNA). The polynucleotide-guided effector protein may contain one nuclease domain that cuts one or both strands of a double stranded polynucleotide, or may contain two nuclease domains wherein a first nuclease domain is positioned for cleavage of one strand of the target polynucleotide and a second nuclease domains is positioned for cleavage of the complementary strand of the target polynucleotide. The nuclease domains may be active or inactive. For example, the nuclease domain or one or both of the two nuclease domains may be inactivated by mutation.

The guide polynucleotide may be a guide RNA, a guide DNA, or a guide containing both DNA and RNA. The guide polynucleotide is preferably a guide RNA. Therefore, the polynucleotide-guided effector protein is preferably an RNA-guided effector protein.

The RNA-guided effector protein may be any protein that binds to or is attached to the guide RNA. The RNA-guided effector protein typically binds to a region of guide RNA that is not the region of guide RNA which binds to the target polynucleotide. For example, where the guide RNA comprises crRNA and tracrRNA, the RNA-guided effector protein typically binds to the tracrRNA and the crRNA typically binds to the target polymer, such as target polynucleotide. The RNA-guided effector protein preferably also binds to a target polynucleotide. The region of the guide RNA that binds to the target polynucleotide may also bind to the RNA-guided effector protein. The RNA-guided effector protein typically binds to a double stranded region of the target polynucleotide. The region of the target polynucleotide to which the RNA-guided effector protein binds is typically located close to the sequence to which the guide RNA hybridizes. The guide RNA and RNA-guided effector protein typically form a complex, which complex then binds to the target polynucleotide at a site determined by the sequence of the guide RNA.

The RNA-guided effector protein may bind upstream or downstream of the sequence to which the guide RNA binds. For example, the RNA-guided effector protein may bind to a protospacer adjacent motif (PAM) in DNA located next to the sequence to which the guide RNA binds. A PAM is a short (less than 10, typically a 2 to 6 base pair) sequence, such as 5'-NGG-3' (wherein N is any base), 5'-NGA-3', 5'-YG-3' (wherein Y is a pyrimidine), 5'TTN-3' or 5'-YTN-3'. Different RNA-guided effector proteins bind to different PAMs. RNA-guided effector proteins may bind to a target polynucleotide which does not comprise a PAM, in particular, where the target is RNA or a DNA/RNA hybrid.

The RNA-guided effector protein is typically a nuclease, such as an RNA-guided endonuclease. The RNA-guided effector protein is typically a Cas protein. The RNA-guided effector protein may be Cas, Csn2, Cpf1, Csf1, Cmr5, Csm2, Csy1, Cse1 or C2c2. The Cas protein may be Cas3, Cas 4, Cas8a, Cas8b, Cas8c, Cas9, Cas10 or Cas10d. Preferably, the Cas protein is Cas9. Cas, Csn2, Cpf1, Csf1, Cmr5, Csm2, Csy1 or Cse1 is preferably used where the target polynucleotide comprises a double stranded DNA region. C2c2 is preferably used where the target polynucleotide comprises a double stranded RNA region.

A DNA-guided effector protein, such as proteins from the RecA family may be used to target DNA. Examples of proteins from the RecA family that may be used are RecA, RadA and Rad51. The nuclease activity of the RNA-guided endonuclease may be disabled. One or more of the catalytic nuclease sites of the RNA-guided endonuclease may be inactivated. For example, where the RNA-guided endonuclease comprises two catalytic nuclease sites, one or both of the catalytic sites may be inactivated. Typically, one of the catalytic sites will cut one strand of the polynucleotide to which it specifically binds and the other catalytic site will cut the opposite strand of the polynucleotide. Therefore, the RNA-guided endonuclease may cut both strands, one strand or neither strand of a double stranded region of a target polynucleotide.

The polynucleotide-guided effector protein is preferably Cas9. Cas9 has a bi-lobed, multi-domain protein structure comprising target recognition and nuclease lobes. The recognition lobe binds guide RNA and DNA. The nuclease lobe contains the HNH and RuvC nuclease domains which are positioned for cleavage of the complementary and non-complementary strands of the target DNA. The structure of Cas 9 is detailed in Nishimasu, H., et al., (2014) Crystal Structure of Cas9 in Complex with Guide RNA and Target DNA. Cell 156, 935-949. The relevant PDB reference for Cas9 is 5F9R (Crystal structure of catalytically-active Streptococcus pyogenes CRISPR-Cas9 in complex with single-guided RNA and doublestranded DNA primed for target DNA cleavage).

The Cas9 may be an 'enhanced specificity' Cas9 that shows reduced off-target binding compared to wild-type Cas9. An example of such an 'enhanced specificity' Cas9 is S. *pyogenes* Cas9 D10A/H840A/K848A/K1003A/R1060A. ONLP12296 is the amino acid sequence of *S*. *pyogenes* Cas9 D10A/H840A/K848A/K1003A/R1060A having a C-terminal Twin-Strep-tag with TEV-cleavable linker.

Catalytic sites of an RNA-guided endonuclease may be inactivated by mutation. The mutation may be a substitution, insertion, or deletion mutation. For example, one or more, such as 2, 3, 4, 5, or 6 amino acids may be substituted or inserted into or deleted from the catalytic site. The mutation is preferably a substitution insertion, more preferably substitution if a single amino acid at the catalytic site. The skilled person will be readily able to identify the catalytic sites of an RNA-guided endonuclease and mutations that inactivate them. For example, where the RNA-guided endonuclease is Cas9, one catalytic site may be inactivated by a mutation at D10 and the other by a mutation at H640.

An inactivated ('dead') polynucleotide-guided effector protein that does not cut the target polynucleotide and so shows no directionality bias. An active ('live') polynucleotide-guided effector protein that cuts the target polynucleotide may remain bound to just one of the two ends of the cut site and so may show some directionality bias.

The polymer-guided effector protein typically remains bound to the target polymer for a prolonged period. The polymer-guided effector protein preferably remains bound to the target polymer for from at least about 1 to at least about 10, such as about 2 to about 8 hours or about 4 to about 6 hours.

The polymer-guided effector protein typically remains bound to the target polymer, if present, and translocates through the pore as part of the complex. The translocation of the protein produces an electrical or current effect that is measured in accordance with the invention and provides information. This is discussed in more detail below.

The polymer-guided effector protein may be specifically modified for use in a method of the invention. The protein may comprise an anchor capable of coupling to a membrane, such as cholesterol. The polymer-guided effector protein preferably has a binding moiety capable of coupling to a surface attached thereto. The surface is preferably the surface of beads.

### Guide polymer

The guide polymer is capable of binding to a target polymer and mediating the binding of the polymer-guided effector protein to the target polymer. The guide polymer may have any structure that enables it to bind to the target polymer. It may be any of the polymers discussed above with reference to the target polymer. The guide polymer is preferably an oligonucleotide, a polynucleotide, a polypeptide, a protein, an oligosaccharide, or a polysaccharide. The guide polypeptide or protein is preferably a zinc finger binding protein, a transcription activator-like effector (TALE), transcription factor, restriction enzyme, DNA-binding protein or enzyme, an antibody, or an antibody fragment. Suitable antibody fragments are known in the art and include, but are not limited to, Fab, Fab', (Fab')2, FV, scFv, diabody, triabody, tetrabody, Bis-scFv, minibody, Fab2 and Fab3. The guide oligonucleotide is preferably an aptamer.

The guide polymer may bind to the polymer-guided effector protein. In this context, the guide polymer may bind to polymer-guided effector protein, may be bound by the polymer-guided effector protein or both. The guide polymer may be attached to the polymer-guided effector protein. Suitable methods are known in the art for attaching the guide polymer to the polymer-guided effector protein, for instance using streptavidin/biotin. The polymer-guided effector is preferably covalently attached to the guide polymer. Covalent attachment, for instance using click chemistry, is discussed in WO 2018/060740.

The guide polymer is preferably a guide polynucleotide. In this instance, the polymer-guided effector protein is preferably a polynucleotide-guided effector protein. The guide polynucleotide preferably comprises a sequence that is capable of binding to the target polymer or hybridising to the target polynucleotide. The guide polynucleotide preferably comprises a nucleotide sequence that binds to a sequence in the target polymer and a nucleotide sequence that binds to the polynucleotide-guided effector protein. The guide polynucleotide preferably comprises a nucleotide sequence that hybridises to a sequence in the target polynucleotide and a nucleotide sequence that binds to the polynucleotide-guided effector protein. The guide polynucleotide may have any structure that enables it to bind/hybridise to the target polymer and bind to a polynucleotide-guided effector protein.

If the target polymer is a polynucleotide, the guide polynucleotide typically hybridizes to a sequence of about 20 nucleotides in the target polynucleotide. The sequence to which the guide polynucleotide binds may be from about 10 to about 40, such as from about 15 to about 30, preferably from about 18 to about 25, such as about 19, 20, 21, 22, 23 or 24 nucleotides. The guide polynucleotide is typically complementary to one strand of a double stranded region of the target polynucleotide. The guide polynucleotide preferably comprises a nucleotide sequence of from about 10 to about 40, such as from about 15 to about 30, preferably from about 18 to about 25, such as about 19, 20, 21, 22, 23 or 24, nucleotides that is complementary to the sequence of, or to a sequence in, the target polynucleotide. The degree of complementarity is preferably exact.

The guide polynucleotide is preferably guide RNA. The guide RNA may be complementary to a region in the target polynucleotide that is 5' to a PAM. This is preferred where the target polynucleotide comprises DNA, particularly where the RNA effector protein is Cas9 or Cpf1. The guide RNA may be complementary to a region in the target polynucleotide that is flanked by a guanine. This is preferred where the target polynucleotide comprises RNA, particularly where the RNA effector protein is C2c2.

The guide RNA may have any structure that enables it to bind to the target polynucleotide and to an RNA-guided effector protein. The guide RNA may comprise a crRNA that binds to a sequence in the target polynucleotide and a tracrRNA. The tracrRNA typically binds to the RNA-guided effector protein. Typical structures of guide RNAs are known in the art. For example, the crRNA is typically a single stranded RNA and the tracrRNA typically has a double stranded region of which one strand is attached to the 3' end of the crRNA and a part that forms a hairpin loop at the 3' end of the strand that is not attached to the crRNA. The crRNA and tracrRNA may be transcribed *in vitro* as a single piece sgRNA. The guide RNA is preferably a sgRNA

The guide RNA may comprise other components, such as additional RNA bases or DNA bases or other nucleobases. The RNA and DNA bases in the guide RNA may be natural bases or modified bases. A guide DNA may be used in place of a guide RNA, and a DNA-guided effector protein used instead of an RNA-guided effector protein. The used of a guide DNA and a DNA-guided effector protein may be preferred where the target polynucleotide is RNA.

The guide polymer may be specifically modified for use in a method of the invention. The method may use a guide polymer, particularly a guide RNA, that comprises (i) an adaptor sequence, optionally including a barcode and/or leader sequence and/or (ii) an anchor capable of coupling to a membrane. The anchor is preferably cholesterol. The anchor is preferably attached to the guide polymer, such as guide polynucleotide, via a polynucleotide hybridized to an extension on the guide polymer. The target polymer preferably has an anchor capable of coupling to a membrane attached thereto and the guide polymer preferably has an adaptor and/or leader sequence attached thereto

The guide polymer preferably has a binding moiety capable of coupling to a surface attached thereto. The surface is the surface of beads. The binding moiety is preferably an end extension on the guide polynucleotide. The guide polymer preferably comprises two binding moieties, which are optionally end extensions on the guide polynucleotide.

The guide polymer may be any of the guide polymers discussed herein to which (i) an adaptor and/or (ii) an anchor capable of coupling to a membrane is attached.

The (i) the anchor or (ii) the adaptor may be present at the 5' end of the tracrRNA, the 3' end of the tracrRNA, the 3' end of the crRNA, or internally, for example, wherein the tracrRNA and crRNA are comprised in a sgRNA. The (i) the anchor or (ii) the adaptor may be added to the 5' end of the crRNA, *e*.*g*., via a chemical group or spacer. The (i) the anchor or (ii) the adaptor may be added to the guide polymer via a chemical group or spacer. The (i) the anchor or (ii) the adaptor may be attached to the 5' or 3' end of the guide polymer, such as to the 5' or 3' end of a tracrRNA or the 5' or 3' end of a crRNA by any suitable means, *e.g.,* ligation, via a chemical group, *e.g.,* thiols, click groups, biotin etc., or via a DNA, RNA, PNA, BNA, LNA, TNA spacer. Where the spacer is a polynucleotide, the spacer may have a length of from about 1 to about 30, such as from about 2 to about 20, from about 3 to about 15, from about 4 to about 10, such as about 5, 6, 7 8 or 9, nucleotides.

The anchor may be attached to an oligonucleotide (anchor oligonucleotide) that is complementary to an end extension, or an internal loop sequence, in the guide polymer. The 5' end of the tracrRNA, the 3' end of the tracrRNA, the 3' end of the crRNA or the 5' end of the crRNA may have an end extension having a length of, for example, from about 5 to about 40, such as from about 10 to about 30 or from about 15 to about 25 nucleotides, for example about 20 nucleotides. The anchor oligonucleotide may have the same length as the end extension, or the anchor oligonucleotide may be longer or shorter than the end extension, provided that anchor oligonucleotide and end extension have sequences that are complementary over a length of from about 5 to about 40, such as about 10 to about 30 or from about 15 to about 25 nucleotides, for example about 20 nucleotides. The anchor oligonucleotide may have a length of, for example, from about 5 to about 40, such as from about 10 to about 30 or from about 15 to about 25 nucleotides, for example about 20 nucleotides. The internal loop sequence may have any of the lengths specified above.

The guide polymer may be synthetically modified. Both the 5' and 3' ends of crRNA and tracrRNA can be modified. See Lee et al., (2017) Synthetically modified guide RNA and donor DNA are a versatile platform for CRISPR-Cas9 engineering. eLIFE;6:e25312. Synthetic modification may comprise incorporation of modified or artificial bases into guide RNA (or guide DNA), including DNA, RNA, PNA, LNA, BNA, DNA spacers, RNA spacers and abasic spacers *e*.*g*., Sp18. Alternatively modification may comprise modification with chemical moieties that are structurally unrelated to nucleotide bases such as planar hydrophobic molecules, chemical tags, fluorescent molecules, aptamer sequences, amines, azides, alkynes, thiols, click groups, biotins.

If the guide polymer is a guide polynucleotide, the guide polynucleotide may have a polynucleotide binding protein capable of moving along a polynucleotide attached thereto. The polynucleotide binding protein may be bound close to one end of a strand of the guide polynucleotide, typically close to the 5' end. The end to which the polynucleotide binding protein is bound is typically modified by the addition of an adaptor, preferably an adaptor comprising a leader sequence. Where the guide RNA comprises a leader sequence the polynucleotide binding protein is typically bound to the leader sequence.

Where the guide RNA comprises a crRNA, the polynucleotide binding protein may be positioned such that it is capable of moving along the crRNA. Such a guide RNA is useful in the method in which the crRNA translocates through the pore in order to detect the presence or absence of the target polymer.

The guide polymer may be specifically adapted to enable capture of the target polymer on a surface, such as a bead or column. This allows target polymers to which a guide polymer/polymer-guided effector protein complex is bound to be captured and separated from non-target polymers in a sample, which can then be washed away. The guide polymer may comprise an end extension at the 3' or 5' end, which has a sequence that is complementary to the sequence of a capture oligonucleotide that is bound to a surface, such as to a bead or column. For example, the capture oligonucleotide may be bound to the surface by an affinity tag. Any suitable affinity tag may be used. One example is a biotin-streptavidin affinity tag. The capture oligonucleotide may have a length of from about 5 to about 40, such as from about 10 to about 30 or from about 15 to about 25 nucleotides, for example about 20 nucleotides or a length of from about 10 to about 80, such as from about 20 to about 60 or from about 30 to about 50 nucleotides, for example about 40 nucleotides. The end extension may have a length of from about 5 to about 40, such as from about 10 to about 30 or from about 15 to about 25 nucleotides, for example about 20 nucleotides. The capture oligonucleotide may have the same length as the end extension, or the capture oligonucleotide may be longer or shorter than the end extension, provided that capture oligonucleotide and end extension have sequences that are complementary over a length of from about 5 to about 40, such as from about 10 to about 30 or from about 15 to about 25 nucleotides, for example about 20 nucleotides.

The guide polymer may comprise a first binding moiety and a second binding moiety. The first binding moiety and the second binding moiety may both be end extensions, or other single stranded polymer sequences capable of binding to an oligonucleotide, on the guide polymer. The first end extension on the guide polymer may have a sequence complementary to a first capture oligonucleotide on a first capture surface, *e*.*g*., a bead, and the second end extension on the guide polymer may have a sequence complementary to a second capture oligonucleotide on a second capture surface, *e*.*g*., a bead. One way of configuring this the crRNA comprises a 3' DNA extension used for capture of the target molecule on a bead, column, or surface and the tracrRNA comprises a 5' DNA extension. In one embodiment, the first end extension comprises an end portion, which is at the 5' end in a 5' end extension or at the 3' end in a 3' end extension, which has a sequence that is not complementary to a sequence in the first capture oligonucleotide, and a portion that has a sequence that is complementary to the first sequence in the first capture oligonucleotide. The end portion of the first end extension may typically have a length of from about 2 to about 10 nucleotides, such as about 3, 4, 5 or 6 nucleotides. The portion of the first end extension that is complementary to the first capture oligonucleotide may typically have a length of from about 5 to about 40, such as from about 10 to about 30 or from about 15 to about 25 nucleotides, for example about 20 nucleotides.

The second end extension has a sequence that does hybridise to the first capture nucleotide, the first end extension or the competitor oligonucleotide. The second capture oligonucleotide also has a sequence that does hybridise to the first capture nucleotide, the first end extension or the competitor oligonucleotide. The end extension may be attached to the 5' or 3' end of the guide polymer, such as to the 5' or 3' end of a tracrRNA or the 5' or 3' end of a crRNA. The end extension may be attached to the guide polymer by any suitable means. The end extension may, for example, may be added via a chemical group or spacer, *e.g.,* ligation, via a chemical group, *e.g.,* thiols, click groups, biotin etc., or via a DNA, RNA, PNA, BNA, LNA, TNA spacer. Where the spacer is a polynucleotide, the spacer may have a length of from about 1 to about 30, such as from about 2 to about 20, from about 3 to about 15, from about 4 to about 10, such as from about 5, 6, 7, 8 or 9, nucleotides. The end extension may be present at the 5' end of the tracrRNA, the 3' end of the tracrRNA, the 3' end of the crRNA, the 5' end of the crRNA or may be substituted by a sequence added internally to the guide RNA, for example, wherein the tracrRNA and crRNA are comprised in a sgRNA. Where an internal sequence is used to perform the function described herein for the end extension, it is typically present in a loop structure within the guide polymer, or is otherwise accessible for hybridization to the capture oligonucleotide.

### Sample

The sample may be any suitable sample. The sample is typically one that is known to contain or is suspected of containing at least one of the target polymers. The method can be used to select target polymers for delivery to the pore. Other components of the sample may be washed away, for example, they may be flushed out of a cell comprising the pore. Such other components include one or more of the following: proteins, which may be folded or unfolded, peptides, carbohydrates, polymers, such as non-target polymers, and cell debris.

The sample may be a biological sample. The invention may be carried out *in vitro* on a sample obtained from or extracted from any organism or microorganism. The organism or microorganism is typically archaean, prokaryotic or eukaryotic and typically belongs to one the five kingdoms: plantae, animalia, fungi, monera and protista. The invention may be carried out *in vitro* on a sample obtained from or extracted from any virus.

The sample is preferably a fluid sample. The sample typically comprises a body fluid. The body fluid may be obtained from a human or animal. The human or animal may have, be suspected of having or be at risk of a disease. The sample may be urine, lymph, saliva, mucus, seminal fluid, or amniotic fluid, but is preferably whole blood, plasma, or serum. Typically, the sample is human in origin, but alternatively it may be from another mammal such as from commercially farmed animals such as horses, cattle, sheep, or pigs or may alternatively be pets such as cats or dogs.

Alternatively, a sample of plant origin is typically obtained from a commercial crop, such as a cereal, legume, fruit, or vegetable, for example wheat, barley, oats, canola, maize, soya, rice, bananas, apples, tomatoes, potatoes, grapes, tobacco, beans, lentils, sugar cane, cocoa, cotton, tea, or coffee.

The sample may be a non-biological sample. The non-biological sample is preferably a fluid sample. Examples of non-biological samples include surgical fluids, water such as drinking water, sea water or river water, and reagents for laboratory tests.

The sample may be processed prior to being assayed, for example by centrifugation or by passage through a membrane that filters out unwanted molecules or cells, such as red blood cells. The sample may be measured immediately upon being taken. The sample may also be typically stored prior to assay, preferably below -70°C.

The sample may comprise genomic DNA. The genomic DNA may be fragmented or step (a) of the method may further comprise fragmenting the genomic DNA. The DNA may be fragmented by any suitable method. For example, methods of fragmenting DNA are known in the art. Such methods may use a transposase, such as a MuA transposase.

The sample may comprise T-cell DNA.

The sample may comprise non-target polymers. In one embodiment, the target polynucleotide and the non-target polynucleotides may be derived from the same gene or genome.

### Electrical measurements and effects

Step (c) comprises applying a potential across the membrane. The applied potential may be a voltage potential. Alternatively, the applied potential may be a chemical potential. An example of this is using a salt gradient across a membrane, such as any of the membranes discussed below. A salt gradient is disclosed in Holden et al., J Am Chem Soc. 2007 Jul 11; 129(27):8650-5. In some instances, the current passing through the pore as a polynucleotide moves with respect to the pore is used to estimate or determine the sequence of the polynucleotide. This is strand sequencing.

Step (c) also comprises taking one or more electrical measurements. Suitable electrical measurements include, but are not limited to, a current measurement, a conductance measurement, a capacitance measurement, an impedance measurement, a tunnelling measurement (Ivanov AP et al., Nano Lett. 2011 Jan 12; 11(1):279-85), and a FET measurement (International Application WO 2005/124888). Any number and combination of these measurements may be made in the method. The one or more electrical measurements may comprise a transmembrane current measurement, such as measurement of ionic current flowing through the pore.

One or more optical measurements may be combined with the one or more electrical measurements (Soni GV et al., Rev Sci Instrum. 2010 Jan; 81(1):014301). The one or more optical measurements may be those described in Chen et al., Nat Commun 9, 1733 (2018).

Electrical measurements may be made using standard single channel recording equipment as describe in Stoddart D et al., Proc Natl Acad Sci, 12;106(19):7702-7, Lieberman KR et al, J Am Chem Soc. 2010;132(50):17961-72, and International Application WO 2000/28312. Alternatively, electrical measurements may be made using a multichannel system, for example as described in International Application WO 2009/077734 and International Application WO 2011/067559.

Step (c) preferably comprises applying a potential difference across the membrane and measuring the current flowing through the pore. Step (d) preferably comprises monitoring for the presence or absence of a current effect resulting from the translocation of the complex through the pore and thereby determining the presence or absence of the target polymer in the sample.

Step (d) comprises monitoring for the presence or absence of an electrical effect, preferably a current effect, resulting from the translocation of the complex through the pore and thereby determining the presence or absence of the target polymer in the sample. The effect is indicative of the complex formed by the guide polynucleotide, the polynucleotide-guided effector protein and the target polynucleotide interacting with the pore as it translocates. If an electrical or current effect associated with only the guide polymer and the polymer-guided effector protein translocating through the pore is seen, the target polymer is absent from the sample. If an electrical or current effect associated with the complex (*i.e.*, the guide polymer, the polymer-guided effector protein, and the target polymer) translocating through the pore is seen, the target polymer is present in the sample. The difference between the two effects is discussed below.

The effect may also be caused by the translocation through the pore of an adaptor attached to one of the components of the complex, the target polynucleotide, or the guide polynucleotide. In this instance, the effect is indicative of the translocation through the pore of an adaptor attached to one of the components of the complex, the target polynucleotide, or the guide polynucleotide. The method preferably comprises determining the presence or absence of the interaction of the adaptor with the pore. The adaptor preferably comprises a barcode.

The effect is monitored using the one or more electrical measurements. The effect is typically a measured change in one or more aspects of the one or more electrical measurements. The one or more aspects, include but are not limited to, quantity, duration, consistency, and complexity of the one or more electrical measurements. Duration is also known as dwell time because the effect relates to the complex or a part thereof dwelling in the biological pore. Some of these aspects are discussed in more detail below. The effect is preferably a measured change in one or more aspects, such as one or more of quantity, duration, consistency, and complexity, of the current flowing through the pore.

The complex comprises the target polymer, if present, the polymer-guided effector protein and the guide polymer so the electrical or current effect resulting from the translocation of the complex through the pore (as opposed to just the guide polymer and the polymer-guided effector protein) provides information about the presence of the target polymer and information about both the target polymer and the polymer-guided effector protein. The bulky polymer-guided effector protein interacts with the pore and takes up space within the opening of the pore as it translocates and so typically results in a long, pronounced, and complex effect on the one or more electrical measurements, such as current flow. In contrast, the target polymer moves quickly through the pore with fewer interactions and less blocking of the pore opening and so typically has a shorter, less pronounced, and less complex effect on the one or more electrical measurements, such as current flow. These different effects allow the method of the invention to determine whether or not the target polymer is present. The target polymer is absent if only the effect of the polymer-guided effector protein and bound/attached guide polymer is seen. The target polymer is present if the target polymer effect is seen in addition to the effect of the polymer-guided effector protein and bound/attached guide polymer.

Translocation of the polymer-guided effector protein part of the complex through the pore preferably results in an electrical effect that is one or more of (i) longer, (ii) more pronounced and (iii) more complex than the electrical effect associated with the target polymer part of the complex translocating through the pore. Translocation of the polymer-guided effector protein part of the complex through the pore preferably results in an electrical effect that is (i), (ii), (iii), (i) and (ii), (i) and (iii), (ii) and (iii) or (i), (ii) and (iii) than the electrical effect associated with the target polymer part of the complex translocating through the pore. Translocation of the polymer-guided effector protein part of the complex through the pore preferably results in an electrical effect that is (i) longer, (ii) more pronounced and (iii) more complex than the electrical effect associated with the target polymer part of the complex translocating through the pore. In these embodiments, the guide polymer is typically bound/attached to the polymer-guided effector protein as it translocates. The guide polymer may be covalently attached to the polymer-guided effector protein. The effect of the bulky protein typically masks any observable effect of the bound/attached guide polymer.

The effect is longer if the effect resulting from the protein translocating through the pore happens for a longer duration (or more time) than the effect resulting from the target polymer translocating through the pore. The effect is longer if the change in quantity of the one or more electrical measurements resulting from the protein translocating through the pore happens for a longer duration (or more time) than the change in quantity of the one or more electrical measurements resulting from the target polymer translocating through the pore.

The effect is more pronounced if the effect resulting from the protein translocating through the pore is greater than the effect resulting from the target polymer translocating through the pore. The effect is more pronounced if the amount the change in quantity of the one or more electrical measurements resulting from the protein translocating through the pore is greater than the change in quantity of the one or more electrical measurements resulting from the target polymer translocating through the pore.

A more complex effect preferably comprises the effect resulting from the polymer-guided effector protein translocating through the pore (i) being inconsistent, (ii) noisy and (iii) involving stepping events when compared with the effect resulting from the target polymer translocating through the pore. A more complex effect preferably comprises the measured change in one or more aspects of the one or more electrical measurements resulting from the polymer-guided effector protein translocating through the pore (i) being inconsistent, (ii) noisy and (iii) involving stepping events when compared with the measured change in one or more aspects of the one or more electrical measurements resulting from the target polymer translocating through the pore. The more complex effect may comprise (i), (ii), (iii), (i) and (ii), (i) and (iii), (ii) and (iii) or (i), (ii) and (iii).

Step (c) preferably comprises measuring the current flowing through the pore. Translocation of the polymer-guided effector protein part of the complex through the pore preferably results in a current effect that is one or more of (i) longer, (ii) more pronounced and (iii) more complex than the current effect associated with the target polymer part of the complex translocating through the pore. The effect may be any combination of (i)-(iii) as set out above. Translocation of the polymer-guided effector protein part of the complex through the pore more preferably results in a current effect that is (i) longer, (ii) more pronounced and (iii) more complex than the current effect associated with the target polymer part of the complex translocating through the pore.

The current effect is longer if the change in the current resulting from the protein translocating through the pore happens for a longer duration (or more time) than the change in the current resulting from the target polymer translocating through the pore.

The current effect is more pronounced if the change in the current resulting from the protein translocating through the pore is greater than the change in the current resulting from the target polymer translocating through the pore. The polymer-guided effector protein preferably results in an at least about 20% change in the open pore current as is translocates through the pore. The polymer-guided effector protein more preferably results in an at least about 30% change, at least about 40% change, at least about 50% change, at least about 60% change, at least about 70% change or at least about 80% change in the open pore current as is translocates through the pore. The guide polymer is typically bound/attached to the polymer-guided effector protein as it translocates. The target polymer preferably results in an about 10% or lower change in the open pore current as is translocates through the pore. The target polymer preferably results in an about 9% or lower change, about 8% or lower change, about 7% or lower change, about 6% or lower change or about 5% or lower change in the open pore current as is translocates through the pore.

A more complex current effect comprises preferably one or more of (i) an inconsistent effect, (ii) noise and (iii) electrical stepping events. In these embodiments, the guide polymer is typically bound/attached to the polymer-guided effector protein as it translocates. The guide polymer may be covalently attached to the polymer-guided effector protein. The effect of the bulky protein typically masks any observable effect of the bound/attached guide polymer. The effect may be any combination of (i)-(iii) as set out above.

If one guide polymer and one polymer-guided effector protein are used to form one complex, the electrical or current effect associated with the target polymer is preferably observed before and/or after the electrical or current effect associated with the polymer-guided effector protein. The timing will depend on where the guide polymer binds to the target polymer. If the guide polymer binds/the complex forms at or towards one end of the target polymer, the electrical or current effect associated with the target polymer is preferably observed before or after the electrical or current effect associated with the polymer-guided effector protein and guide polymer. Whether it's before or after depends on whether the free end of the target polynucleotide (before) or the end with the guide polymer-guided effector protein and guide polymer bound (after) enters the pore first.

If two or more guide polymers and two or more polymer-guided effector proteins are used to form two or more complexes, the electrical or current effects associated with the target polymer part of the two or more complexes translocating through the pore may be observed before and/or after the electrical or current effects resulting from the polymer-guided effector protein parts of the two or more complexes translocating through the pore. The electrical or current effects associated with the target polymer part of the two or more complexes translocating through the pore may be observed between the electrical or current effects resulting from the polymer-guided effector protein parts of the two or more complexes translocating through the pore. For instance, if two guide polymers and two polymer-guided effector proteins are used such that complexes form at each end of the target polymer, the effect of one complex with be seen, followed by the effect of the target polymer, followed by the effect of the second complex. Any number of guide polymer/ polymer-guided effector protein combinations may be used to bind to different parts of the target polymer and provide a different combination of observable electrical or current effects. The electrical or current effects associated with the target polymer translocating through the pore may be observed (a) before and/or after and (b) between two or more electrical or current effects resulting from the two or more polymer-guided effector proteins translocating through the pore.

The methods may involve measuring the current passing through the pore as the polymer moves with respect to the pore. Therefore, the apparatus may also comprise an electrical circuit capable of applying a potential and measuring an electrical signal across the membrane and pore. The methods may be carried out using a patch clamp or a voltage clamp. The methods preferably involve the use of a voltage clamp.

The methods may involve the measuring of a current passing through the pore as the polymer moves with respect to the pore. Suitable conditions for measuring ionic currents through protein pores are known in the art and disclosed in the Example. The method is typically carried out with a voltage applied across the membrane and pore. The voltage used is typically from +5 V to -5 V, such as from +4 V to -4 V, +3 V to -3 V or +2 V to -2 V. The voltage used is typically from -600 mV to +600mV or -400 mV to +400 mV. The voltage used is preferably in a range having a lower limit selected from -400 mV, -300 mV, -200 mV, -150 mV, -100 mV, -50 mV, -20mV and 0 mV and an upper limit independently selected from +10 mV, + 20 mV, +50 mV, +100 mV, +150 mV, +200 mV, +300 mV and +400 mV. The voltage used is more preferably in the range 100 mV to 240 mV and most preferably in the range of 120 mV to 220 mV. It is possible to increase discrimination between different nucleotides by a pore by using an increased applied potential.

The methods are typically carried out in the presence of any charge carriers, such as metal salts, for example alkali metal salt, halide salts, for example chloride salts, such as alkali metal chloride salt. Charge carriers may include ionic liquids or organic salts, for example tetramethyl ammonium chloride, trimethylphenyl ammonium chloride, phenyltrimethyl ammonium chloride, or 1-ethyl-3-methyl imidazolium chloride. In the exemplary apparatus discussed above, the salt is present in the aqueous solution in the chamber. Potassium chloride (KCI), sodium chloride (NaCl), caesium chloride (CsCl) or a mixture of potassium ferrocyanide and potassium ferricyanide is typically used. KCI, NaCl and a mixture of potassium ferrocyanide and potassium ferricyanide are preferred. The charge carriers may be asymmetric across the membrane. For instance, the type and/or concentration of the charge carriers may be different on each side of the membrane.

The salt concentration may be at saturation. The salt concentration may be 3 M or lower and is typically from 0.1 to 2.5 M, from 0.3 to 1.9 M, from 0.5 to 1.8 M, from 0.7 to 1.7 M, from 0.9 to 1.6 M or from 1 M to 1.4 M. The salt concentration is preferably from 150 mM to 1 M. The method is preferably carried out using a salt concentration of at least 0.3 M, such as at least 0.4 M, at least 0.5 M, at least 0.6 M, at least 0.8 M, at least 1.0 M, at least 1.5 M, at least 2.0 M, at least 2.5 M or at least 3.0 M. High salt concentrations provide a high signal to noise ratio and allow for currents indicative of the presence of a nucleotide to be identified against the background of normal current fluctuations.

The methods are typically carried out in the presence of a buffer. In the exemplary apparatus discussed above, the buffer is present in the aqueous solution in the chamber. Any buffer may be used in the method of the invention. Typically, the buffer is phosphate buffer. Other suitable buffers are HEPES and Tris-HCI buffer. The methods are typically carried out at a pH of from 4.0 to 12.0, from 4.5 to 10.0, from 5.0 to 9.0, from 5.5 to 8.8, from 6.0 to 8.7 or from 7.0 to 8.8 or 7.5 to 8.5. The pH used is preferably about 7.5.

The methods may be carried out at from 0 °C to 100 °C, from 15 °C to 95 °C, from 16 °C to 90 °C, from 17 °C to 85 °C, from 18 °C to 80 °C, 19 °C to 70 °C, or from 20 °C to 60 °C. The methods are typically carried out at room temperature. The methods are optionally carried out at a temperature that supports enzyme function, such as about 37 °C.

### Pore

The method of the invention uses a biological pore with opening or channel that permits translocation of the complex through the pore. The minimum diameter of the pore opening or channel is large enough to permit translocation of the complex through the pore. The pore may have a constriction in the opening or channel that permits translocation of the complex through the pore. The opening or channel preferably permits translocation of the complex through the pore and allows one or more interactions between the pore and the polymer-guided effector protein. The minimum diameter of the pore opening or channel is large enough to permit translocation of the complex through the pore and allows one or more interactions between the pore and the polymer-guided effector protein. The pore may have a constriction in the opening or channel that permits translocation of the complex through the pore and allows one or more interactions between the pore and the polymer-guided effector protein. The one or more interactions may be selected from physical interactions, steric interactions, electrostatic interactions, hydrogen bonding and Van der Waals interactions.

The opening or channel is preferably at least about 5nm in diameter, such as at least about 5.5nm, at least about 6nm, at least about 7nm, at least about 8nm, at least about 9nm, least about 10nm, at least about 11nm or at least about 12nm in diameter. The opening or channel is typically less than about 20 nm in diameter but can be up to about 100nm in diameter. The opening or channel is preferably from about 5nm to about 20nm in diameter, such from about 6nm to about 19nm, from about 7nm to about 18nm, from about 8nm to about 17 nm, from about 9nm to about 16nm or from about 10nm to about 15nm in diameter. The opening or channel is preferably from about 11nm to about 13nm, such as about 12nm, in diameter if the polymer-guided effector protein is an RNA-guided endonuclease, such as Cas, Cas12a (formerly known as Cpf1) or C2c2. The opening or channel is preferably from about 11nm to about 13nm, such as about 12nm, in diameter if the polymer-guided effector protein is a Cas9. The largest dimension of the polymer-guided effector protein is preferably about 3nm or less, such as about 2nm or less or about 1nm or less, smaller than the opening or channel of the biological pore.

The biological pore is a structure that crosses the membrane to some degree. It permits hydrated ions driven by an applied potential to flow across or within the membrane. The pore typically crosses the entire membrane so that hydrated ions may flow from one side of the membrane to the other side of the membrane. However, the pore does not have to cross the membrane. It may be closed at one end. For instance, the pore may be a well, gap, channel, trench or slit in the membrane along which or into which hydrated ions may flow.

A biological pore is a pore that is constructed from biological materials, such as polypeptides, proteins, or polynucleotides. The biological pore may be a naturally occurring biological pore or a modified version of a naturally occurring biological pore. Suitable modifications are known in the art and are discussed below.

The biological pore is preferably a protein pore. A protein pore is a polypeptide or a collection of polypeptides that permits hydrated ions, such as polynucleotide, to flow from one side of a membrane to the other side of the membrane. In the present invention, the protein pore is capable of forming a pore that permits hydrated ions driven by an applied potential to flow from one side of the membrane to the other. The protein pore permits polynucleotides to flow from one side of the membrane, such as a triblock copolymer membrane, to the other. The protein pore allows a polynucleotide, such as DNA or RNA, to be moved through the pore. The pore permits the complex to flow from one side of the membrane, such as a triblock copolymer membrane, to the other.

The protein pore may comprise one or more post-translation modifications (PTMs). The one or more PTMs may be selected from phosphorylation, glycosylation, lipidation, and the formation of disulfide bonds. The one or more PTMs may be modified compared with the wild-type or naturally occurring pore. For example, one or more different amino acids in the protein pore may phosphorylated. The protein pore may lack one of more of these PTMs.

The protein pore is preferably glycosylated. The glycosylation of the protein pore may be modified. The glycosylation of the protein pore is preferably modified compared with the wild-type or naturally occurring pore. The glycosylation may be modified in any manner. The modified glycosylation may comprise (a) one or more different amino acids in the protein pore being glycosylated, (b) the nature of one or more glycans being changed, (c) the type of glycosylation being changed or any combination thereof, such as (a), (b), (c), (a) and (b), (a) and (c), (b) and (c), or (a), (b) and (c). One or more different amino acids in the protein pore being glycosylated includes, but is not limited to, one or more amino acids that are typically glycosylated in the wild-type or naturally occurring pore not being glycosylated, one or more amino acids that are not typically glycosylated in the wild-type or naturally occurring pore being glycosylated and/or one or more glycans being moved to different amino acids. The type of glycosylation may be changed from N-linked glycosylation to O-linked glycosylation or *vice versa.* The protein pore is preferably not glycosylated (also known as aglycosylated) or preferably lacks glycosylation.

The protein pore may be a monomer or an oligomer. The pore is preferably made up of several repeating subunits, such as at least 6, at least 7, at least 8, at least 9, at least 10, at least 11, at least 12, at least 13, at least 14, at least 15, or at least 16 subunits. The pore is preferably a hexameric, heptameric, octameric or nonameric pore. The pore may be a homo-oligomer or a hetero-oligomer. Complement component 9 (C9) is typically a 22-mer.

The protein pore typically comprises a barrel or channel through which the ions may flow. The subunits of the pore typically surround a central axis and contribute strands to a transmembrane β barrel or channel or a transmembrane α-helix bundle or channel.

The barrel or channel of the protein pore typically comprises amino acids that facilitate interaction with the polymer-guided effector protein, as well as nucleotides, polynucleotides, or nucleic acids. These amino acids are preferably located near a constriction of the barrel or channel, if present. The protein pore typically comprises one or more positively charged amino acids, such as arginine, lysine or histidine, or aromatic amino acids, such as tyrosine or tryptophan. These amino acids typically facilitate the interaction between the pore and the polymer-guided effector protein, as well as nucleotides, polynucleotides, or nucleic acids.

The protein pore is preferably Complement component 9 (C9), Perfringolysin O, Pleurotolysin, Listeriolysin, Perforin-2, Gasdermin-A3, L-, P- and M-ring protein, Type II secretion system protein D, GspD, InvG, VirB7, SpoIIIAG, Cag8, Cag3, Cag or other proteins in the Type IV secretion system apparatus protein CagY, WzzB, Pentraxin, Afp2, Major vault protein, Thioredoxin-dependent peroxidase reductase, Arf-GAP, Respiratory syncytial virus ribonucleoprotein, Chikungunya virus nonstructural protein 1, PRC, YaxA, XaxA or PrgH. The pore is preferably C9 if the polymer-guided effector protein is an RNA-guided endonuclease, such as Cas, Cas12a (formerly known as Cpf1) or C2c2. The pore is preferably C9 if the polymer-guided effector protein is Cas9. C9 is an oligomer. In the context of the invention, the C9 pore is an oligomer of the C9 protein monomer.

The biological pore may be an artificial biological pore. Suitable pores are known in the art and include, but are not limited to, DNA origami pores (Langecker et al., Science, 2012; 338: 932-936) and polymer-modified solid-state pores. The solid-state pore may be modified with any of the polymers discussed above.

The biological pore is present in a membrane. The membrane is preferably an amphiphilic layer, such as triblock copolymer membrane, or a solid-state layer. Membranes are discussed in WO 2018/060740. The biological pore may be a protein pore or an artificial pore, such as a DNA origami pore, in an amphiphilic membrane. The biological pore may be a protein pore or an artificial pore, such as a DNA origami pore, in a solid-state layer.

Solid-state layers can be formed from both organic and inorganic materials including, but not limited to, microelectronic materials, insulating materials such as Si₃N₄, A1₂O₃, and SiO, organic and inorganic polymers such as polyamide, plastics such as Teflon^{®} or elastomers such as two-component addition-cure silicone rubber, and glasses. The solid-state layer may be formed from graphene. Suitable graphene layers are disclosed in WO 2009/035647. Yusko et al., Nature Nanotechnology, 2011; 6: 253-260 and US Patent Application No. 2013/0048499 describe the delivery of proteins to pores in solid-state layers without the use of microparticles.

Any of the proteins described herein, such as the protein pores, may be modified to assist their identification or purification, for example by the addition of histidine residues (a his tag), aspartic acid residues (an asp tag), a streptavidin tag, a flag tag, a SUMO tag, a GST tag or a MBP tag, or by the addition of a signal sequence to promote their secretion from a cell where the polypeptide does not naturally contain such a sequence. An alternative to introducing a genetic tag is to chemically react a tag onto a native or engineered position on the pore or construct. An example of this would be to react a gel-shift reagent to a cysteine engineered on the outside of the pore. This has been demonstrated as a method for separating hemolysin hetero-oligomers (Chem Biol. 1997 Jul;4(7):497-505).

The pore may be labelled with a revealing label. The revealing label may be any suitable label which allows the pore to be detected. Suitable labels include, but are not limited to, fluorescent molecules, radioisotopes, *e*.*g*., ¹²⁵I, ³⁵S, enzymes, antibodies, antigens, polynucleotides, and ligands such as biotin.

Any of the proteins described herein, such as the protein pores, may be made synthetically or by recombinant means. For example, the pore may be synthesised by in vitro translation and transcription (IVTT). The amino acid sequence of the pore may be modified to include non-naturally occurring amino acids or to increase the stability of the protein. When a protein is produced by synthetic means, such amino acids may be introduced during production. The pore may also be altered following either synthetic or recombinant production.

Any of the proteins described herein, such as the protein pores, can be produced using standard methods known in the art. Polynucleotide sequences encoding a pore or construct may be derived and replicated using standard methods in the art. Polynucleotide sequences encoding a pore or construct may be expressed in a bacterial host cell using standard techniques in the art. The pore may be produced in a cell by *in situ* expression of the polypeptide from a recombinant expression vector. The expression vector optionally carries an inducible promoter to control the expression of the polypeptide. These methods are described in Sambrook, J. and Russell, D. (2001). Molecular Cloning: A Laboratory Manual, 3rd Edition. Cold Spring Harbor Laboratory Press, Cold Spring Harbor, NY.

The pore may be produced in large scale following purification by any protein liquid chromatography system from protein producing organisms or after recombinant expression. Typical protein liquid chromatography systems include FPLC, AKTA systems, the Bio-Cad system, the Bio-Rad BioLogic system, and the Gilson HPLC system.

### Apparatus, conditions, and arrays

The method may be carried out using the apparatus and conditions described in WO 2008/102120 and/or WO 2018/060740.

The membrane is typically part of an array of membranes, wherein each membrane preferably comprises a biological pore. Therefore, the invention provides a method of detecting a target polymer using an array of membranes. The array may be any of those described in WO 2018/060740.

### Additional embodiments

The adaptor, coupling, linker, anchor, leader sequence, sequencing adaptor or Y adaptor, hairpin loop, bead or microparticle, membrane, array, polynucleotide binding protein and associated free nucleotides or co-factors may be any of those described in WO 2018/060740.

The anchor may be one of those described in Jones et al., J. Am. Chem. Soc. 2021, 143, 22, 8305-8313. The anchor is preferably cholesterol.

### Diagnosis

The methods of the invention can be used to diagnose or prognose a disease or condition. The disease or condition is preferably cancer, coronary heart disease, cardiovascular disease, tuberculosis, or sepsis. Examples of the disease or condition are set out in WO 2018/060740.

Since in an embodiment using a multiplex method the presence of absence of two or more target polymers (*e*.*g*., at least 5 more, 10 or more, 20 or more or 30 or more) may be determined, it is possible to prognose or diagnose two or more (*e*.*g*., 3, 4, 5, 6 or more) of any of the diseases listed above. Accordingly, a multiplex method for detecting and/or analysing a plurality (*e*.*g*., at least 2 or more, at least 3 or more, at least 10 or more, at least 20 or more or at least 30 or more) of target polymers is provided.

The method may also be used to detect polymers, such as polynucleotides, derived from a microorganism or group of microorganisms. This is useful in disease diagnosis and monitoring, but also has other applications. For example, the method may be used to analyse gut or vaginal flora, microorganisms present on the skin or elsewhere. The microorganism may, for example, be a bacterium, virus, fungus, or mycobacterium. The method may be used to determine which infectious agent is causing a disease and hence to determine the best course of treatment. For example, urinary tract infections and other infections are increasingly developing antibacterial resistance. The method may be used to determine the bacterium responsible for an infection and hence to identify an antibiotic or other treatment that will successfully treat the infection.

The method may be used to characterize genomic DNA. In one particular exemplary embodiment, the method may be used to identity polymorphisms, such as SNPs. In another embodiment the method so the invention may be used for repertoire analysis, for example of V(D)J regions. Such methods may use samples derived from blood cells, or T-cells for analysis.

The methods may also be used to characterize unknown polymer sequences.

### System

The invention also provides a system for conducting the methods of the invention. The system is for determining the presence or absence of a target polymer in a sample. In the context of the invention, the terms "system" and "kit" are interchangeable. The invention therefore provides a kit for determining the presence or absence of a target polymer in a sample.

The system or kit comprises a guide polymer that (i) binds to a part of the target polymer if present and (ii) binds to or is attached to a polymer-guided effector protein, wherein the guide polymer and polymer-guided effector protein form a complex with any target polymer present in the sample. Any of the embodiments discussed above with reference to the method of the invention equally apply to the system of the invention.

The system or kit also comprises a biological pore with an opening that permits translocation of the complex through the pore. Any of the pores discussed with reference to the methods of the invention may be present in the system of the invention.

The pore is preferably present in a membrane. Suitable membranes are discussed above. The system or kit may any of the membranes disclosed above, such as an amphiphilic layer, a triblock copolymer membrane or a solid-state layer.

The system or kit is preferably adapted to apply a voltage across the membrane and to take one or more electrical measurements. Suitable adaptations are discussed in WO 2018/060740.

The system or kit may further comprise a polynucleotide binding protein capable of moving along a polynucleotide and/or a leader sequence. The kit may further comprise a microparticle.

The guide polymer, polymer-guided effector protein, anchor, adaptor, polynucleotide binding protein, leader sequence and/or microparticle may be any of those defined herein. The system or kit may comprise a panel of guide polymers or of guide polymer/ polymer-guided effector protein complexes. The panel is typically designed for a particular purpose, such as to detect a particular microorganism, markers associated with a disease, particular polymorphisms etc.

The system or kit may additionally comprise one or more other reagents or instruments which enable any of the embodiments mentioned above to be carried out. Such reagents or instruments include one or more of the following: suitable buffer(s) (aqueous solutions), means to obtain a sample from a subject (such as a vessel or an instrument comprising a needle), means to amplify and/or express polynucleotides, a membrane as defined above or voltage or patch clamp apparatus. Reagents may be present in the system or kit in a dry state such that a fluid sample is used to resuspend the reagents. The system or kit may also, optionally, comprise instructions to enable the system or kit to be used in the methods described herein or details regarding for which organism the method may be used. The system or kit may comprise a magnet or an electromagnet. The system or kit may, optionally, comprise nucleotides.

The system or kit may comprise a guide polymer having an end extension, or first and second end extensions as described herein and a capture oligonucleotide or first and second capture oligonucleotides as described herein. The system or kit may further comprise a competitor oligonucleotide as described herein. The system or kit may further comprise beads comprising one half of an affinity molecule pair (*e*.*g.*, streptavidin) and first and second capture oligonucleotides comprising the other half of an affinity molecule pair (*e*.*g*., biotin). The first and second capture oligonucleotides may each be bound to a separate surface, *e*.*g*., to a separate population of beads. The first capture oligonucleotide may be bound, for example, to "purification" beads or to a "purification" column. The second capture oligonucleotide may be bound, for example, to "delivery" beads. The "purification" beads and/or the "delivery" beads may be magnetic.

The following Example illustrates the invention.

### EXAMPLE

### Materials

The following materials were used:
- dCas9 (Cas9-Spy-(D10A/H840A)): the sequence of this is disclosed in WO 2018/060740
- 3.6kb double stranded linear lambda DNA (SEQ ID NO: 1)
- full length double stranded linear lambda DNA
- tracr RNA (IDT altR)
- CRISPR RNAs (Enlam86, Enlam87, AR837, Enlam51, Enlam5, Enlam26, Enlam45)
- Minion
- C9 monomer
- Oligomerisation buffer (10mM HEPES-NaOH, 50mM NaCl, pH7.5, 0.02mg/mL amphipol)
- Minion flowcell with mediator buffer in cis and trans
- Mediator buffer (300mM potassium ferricyanide, 300mM potassium ferrocyanide, 1M LiCl, 50mM HEPES-NaOH, pH8)
- Binding buffer (25 mM HEPES-NaOH, pH 8.0, 150 mM NaCl, 1 mM MgCl2)
- LiCl buffer (2M LiCl, 25mM HEPES-NaOH, pH8) or any other suitable buffer
- 2x LiCl buffer (4M LiCl, 50mM HEPES-NaOH, pH8) or any other suitable 2x buffer
- Heat block

### C9 oligomerisation protocol

Dilute the C9 monomer to 1mg/ml in oligomerisation buffer and incubate at 37degC for at least 30 minutes and at most 24 hours. This produces oligomerised pore.

### Pore insertion onto minion flowcell

Take oligomerised pore and dilute 1/20,000 in mediator buffer. Add 300uL to the minion flowcell and run the pore insertion script. This script applies a voltage ramp starting at 100mV which it holds for 10 seconds before increasing the voltage by 5mV to 105mV and holding for a further 10 seconds. It continues to increase the voltage by 5mV and hold for 10 seconds at each voltage until it reaches a voltage of 450mV at which point it ends. For the duration of the script the current is monitored in each channel and if a current of >50pA is detected it will switch the channel off and set the voltage to 0. When the script has finished, flush 1mL of mediator buffer through the flowcell. Then flush 1mL of LiCl buffer through the flowcell. This produces LiCl flowcell with pores.

### Preparation of DNA only samples

Dilute 3.6kb double stranded linear lambda DNA to 5nM in LiCl buffer.

### Preparation of DNA samples with a single Cas9 probe attached

1. Snap cool 10uM of tracr RNA by heating to 90degC for 2 minutes and then placing on ice for 10 minutes.
2. In an Eppendorf Protein Lo-Bind tube containing binding buffer add the following components in the following order incubating between steps as noted for a 5uL reaction. Mixing by pipetting after each addition.
   a) 3uM dCas9.
   b) 6uM snap cooled tracr RNA.
   c) Incubate for 5 minutes at room temperature.
   d) 7.5uM CRISPR RNA.
   e) Incubate for 5 minutes at room temperature.
   f) 150nM 3.6kb double stranded linear lambda DNA.
   g) Incubate for at least 20 minutes at room temperature.
3. Take 5uL of the sample prepared in step 2 and add 145uL of LiCl buffer. This is now ready to add to the flowcell.

### Preparation of DNA samples with multiple Cas9 probes attached

1. Snap cool 10uM of tracr RNA by heating to 90degC for 2 minutes and then placing on ice for 10 minutes.
2. In an Eppendorf Protein Lo-Bind tube containing binding buffer add the following components in the following order incubating between steps as noted for a 5uL reaction. Mixing by pipetting after each addition. Perform this step for each probe separately.
   a) 3uM dCas9.
   b) 6uM snap cooled tracr RNA.
   c) Incubate for 5 minutes at room temperature.
   d) 7.5uM CRISPR RNA.
   e) Incubate for 5 minutes at room temperature.
3. In an Eppendorf Protein Lo-Bind tube add 5uL of each probe sample prepared in step 2 above and enough 3.6kb double stranded linear lambda DNA such the concentration in 150uL would be 5nM. The actual concentration in this pot will vary depending on the number of probes. Incubate for at least 20 minutes at room temperature.
4. To the sample prepared in step 3 add 75uL of 2x LiCl buffer and enough water to take the total volume up to 150uL. This is now ready to add to the flowcell.

### Electrophysiology experiments on the minion platform

Assess what the current value is on a LiCl flowcell with pores and if necessary, apply a voltage offset such that it reads as close to 0pA as can be attained. Run an IV curve ramping from 0mV to +- 100mV in 10mV steps holding for 30 seconds at each level (see fig 7). The sampling frequency is set to 30kHz. This serves as the control section before the sample is added.

Add the 150uL sample produced in either of the preceding two sections to the minion flowcell and repeat the IV curve measurement.

### Results

These are shown in Figs. 1-7.

### Sequences

3.6kb double stranded linear lambda DNA (SEQ ID NO: 1) 
GCCATCAGATTGTGTTTGTTAGTCGCTGCCATCAGATTGTGTTTGTTAGTCGCTTTTTTTTTTTGGAAT
TTTTTTTTTGGAATTTTTTTTTTGCGCTAACAACCTCCTGCCGTTTTGCCCGTGCATATCGGTCACGAA
CAAATCTGATTACTAAACACAGTAGCCTGGATTTGTTCTATCAGTAATCGACCTTATTCCTAATTAAAT
AGAGCAAATCCCCTTATTGGGGGTAAGACATGAAGATGCCAGAAAAACATGACCTGTTGGCCGCCAT
TCTCGCGGCAAAGGAACAAGGCATCGGGGCAATCCTTGCGTTTGCAATGGCGTACCTTCGCGGCAG
ATATAATGGCGGTGCGTTTACAAAAACAGTAATCGACGCAACGATGTGCGCCATTATCGCCTAGTTC
ATTCGTGACCTTCTCGACTTCGCCGGACTAAGTAGCAATCTCGCTTATATAACGAGCGTGTTTATCGG
CTACATCGGTACTGACTCGATTGGTTCGCTTATCAAACGCTTCGCTGCTAAAAAAGCCGGAGTAGAA
GATGGTAGAAATCAATAATCAACGTAAGGCGTTCCTCGATATGCTGGCGTGGTCGGAGGGAACTGA
TAACGGACGTCAGAAAACCAGAAATCATGGTTATGACGTCATTGTAGGCGGAGAGCTATTTACTGAT
TACTCCGATCACCCTCGCAAACTTGTCACGCTAAACCCAAAACTCAAATCAACAGGCGCCGGACGCT
ACCAGCTTCTTTCCCGTTGGTGGGATGCCTACCGCAAGCAGCTTGGCCTGAAAGACTTCTCTCCGAA
AAGTCAGGACGCTGTGGCATTGCAGCAGATTAAGGAGCGTGGCGCTTTACCTATGATTGATCGTGG
TGATATCCGTCAGGCAATCGACCGTTGCAGCAATATCTGGGCTTCACTGCCGGGCGCTGGTTATGGT
CAGTTCGAGCATAAGGCTGACAGCCTGATTGCAAAATTCAAAGAAGCGGGCGGAACGGTCAGAGAG
ATTGATGTATGAGCAGAGTCACCGCGATTATCTCCGCTCTGGTTATCTGCATCATCGTCTGCCTGTCA
TGGGCTGTTAATCATTACCGTGATAACGCCATTACCTACAAAGCCCAGCGCGACAAAAATGCCAGAG
AACTGAAGCTGGCGAACGCGGCAATTACTGACATGCAGATGCGTCAGCGTGATGTTGCTGCGCTCG
ATGCAAAATACACGAAGGAGTTAGCTGATGCTAAAGCTGAAAATGATGCTCTGCGTGATGATGTTGC
 
CGCTGGTCGTCGTCGGTTGCACATCAAAGCAGTCTGTCAGTCAGTGCGTGAAGCCACCACCGCCTC
CGGCGTGGATAATGCAGCCTCCCCCCGACTGGCAGACACCGCTGAACGGGATTATTTCACCCTCAG
AGAGAGGCTGATCACTATGCAAAAACAACTGGAAGGAACCCAGAAGTATATTAATGAGCAGTGCAG
ATAGAGTTGCCCATATCGATGGGCAACTCATGCAATTATTGTGAGCAATACACACGCGCTTCCAGCG
GAGTATAAATGCCTAAAGTAATAAAACCGAGCAATCCATTTACGAATGTTTGCTGGGTTTCTGTTTTA
ACAACATTTTCTGCGCCGCCACAAATTTTGGCTGCATCGACAGTTTTCTTCTGCCCAATTCCAGAAAC
GAAGAAATGATGGGTGATGGTTTCCTTTGGTGCTACTGCTGCCGGTTTGTTTTGAACAGTAAACGTC
TGTTGAGCACATCCTGTAATAAGCAGGGCCAGCGCAGTAGCGAGTAGCATTTTTTTCATGGTGTTAT
TCCCGATGCTTTTTGAAGTTCGCAGAATCGTATGTGTAGAAAATTAAACAAACCCTAAACAATGAGTT
GAAATTTCATATTGTTAATATTTATTAATGTATGTCAGGTGCGATGAATCGTCATTGTATTCCCGGATT
AACTATGTCCACAGCCCTGACGGGGAACTTCTCTGCGGGAGTGTCCGGGAATAATTAAAACGATGCA
CACAGGGTTTAGCGCGTACACGTATTGCATTATGCCAACGCCCCGGTGCTGACACGGAAGAAACCG
GACGTTATGATTTAGCGTGGAAAGATTTGTGTAGTGTTCTGAATGCTCTCAGTAAATAGTAATGAATT
ATCAAAGGTATAGTAATATCTTTTATGTTCATGGATATTTGTAACCCATCGGAAAACTCCTGCTTTAGC
AAGATTTTCCCTGTATTGCTGAAATGTGATTTCTCTTGATTTCAACCTATCATAGGACGTTTCTATAAG
ATGCGTGTTTCTTGAGAATTTAACATTTACAACCTTTTTAAGTCCTTTTATTAACACGGTGTTATCGTT
TTCTAACACGATGTGAATATTATCTGTGGCTAGATAGTAAATATAATGTGAGACGTTGTGACGTTTTA
GTTCAGAATAAAACAATTCACAGTCTAAATCTTTTCGCACTTGATCGAATATTTCTTTAAAAATGGCAA
CCTGAGCCATTGGTAAAACCTTCCATGTGATACGAGGGCGCGTAGTTTGCATTATCGTTTTTATCGTT
TCAATCTGGTCTGACCTCCTTGTGTTTTGTTGATGATTTATGTCAAATATTAGGAATGTTTTCACTTAA
TAGTATTGGTTGCGTAACAAAGTGCGGTCCTGCTGGCATTCTGGAGGGAAATACAACCGACAGATGT
ATGTAAGGCCAACGTGCTCAAATCTTCATACAGAAAGATTTGAAGTAATATTTTAACCGCTAGATGAA
GAGCAAGCGCATGGAGCGACAAAATGAATAAAGAACAATCTGCTGATGATCCCTCCGTGGATCTGAT
TCGTGTAAAAAATATGCTTAATAGCACCATTTCTATGAGTTACCCTGATGTTGTAATTGCATGTATAGA
ACATAAGGTGTCTCTGGAAGCATTCAGAGCAATTGAGGCAGCGTTGGTGAAGCACGATAATAATATG
AAGGATTATTCCCTGGTGGTTGACTGATCACCATAACTGCTAATCATTCAAACTATTTAGTCTGTGAC
AGAGCCAACACGCAGTCTGTCACTGTCAGGAAAGTGGTAAAACTGCAACTCAATTACTGCAATGCCC
TCGTAATTAAGTGAATTTACAATATCGTCCTGTTCGGAGGGAAGAACGCGGGATGTTCATTCTTCATC
ACTTTTAATTGATGTATATGCTCTCTTTTCTGACGTTAGTCTCCGACGGCAGGCTTCAATGACCCAGG
CTGAGAAATTCCCGGACCCTTTTTGCTCAAGAGCGATGTTAATTTGTTCAATCATTTGGTTAGGAAAG
CGGATGTTGCGGGTTGTTGTTCTGCGGGTTCTGTTCTTCGTTGACATGAGGTTGCCCCGTATTCAGT
GTCGCTGATTTGTATTGTCTGAAGTTGTTTTTACGTTAAGTTGATGCAGATCAATTAATACGATACCT
GCGTCATAATTGATTATTTGACGTGGTTTGATGGCCTCCACGCACGTTGTGATATGTAGATGATAATC
ATTATCACTTTACGGGTCCTTTCCGGTGAAAAAAAAGGTACCAAAAAAAACATCGTCGTGAGTAGTG
AACCGTAAGC
Enlam86
   An altR cas9 tracr ordered from IDT which will bind to this DNA sequence
   GGGTCATTGAAGCCTGCCGT (SEQ ID NO: 2):
Enlam87
   An altR cas9 tracr ordered from IDT which will bind to this DNA sequence:
   GGTTTCTTCCGTGTCAGCAC (SEQ ID NO: 3)
AR837
   An altR cas9 tracr ordered from IDT which will bind to this DNA sequence:
   TCATGTCTTACCCCCAATAA (SEQ ID NO: 4)
Enlam51
   An altR cas9 tracr ordered from IDT which will bind to this DNA sequence:
   AATCAGCGACACTGAATACG (SEQ ID NO: 5)
Enlam5
   An altR cas9 tracr ordered from IDT which will bind to this DNA sequence:
   CGTGGGCGTACTTTATGGGGCGG (SEQ ID NO: 6)
Enlam26
   An altR cas9 tracr ordered from IDT which will bind to this DNA sequence:
   CGGACTGCGATAATAAGTGGTGG (SEQ ID NO: 7)
Enlam45
   An altR cas9 tracr ordered from IDT which will bind to this DNA sequence:
   TGTGGTAGTGAGATGAAAAGAGG (SEQ ID NO: 8)

## Claims

1. A method of determining the presence or absence of a target polymer in a sample comprising:
a) contacting the sample with a guide RNA that (i) binds to a part of the target polymer and (ii) binds to or is attached to a RNA-guided effector protein, wherein the guide RNA and RNA-guided effector protein form a complex with the target polymer, if present, in the sample;
b) contacting the sample with a membrane comprising a protein pore with an opening that permits translocation of the complex through the pore;
c) applying a potential difference across the membrane and taking one or more electrical measurements; and
d) monitoring for the presence or absence of an electrical effect resulting from the translocation of the complex through the pore and thereby determining the presence or absence of the target polymer in the sample.

2. A method according to claim 1, wherein step (c) comprises applying a potential difference across the membrane and measuring the current flowing through the pore.

3. A method according to claim 1 or 2, wherein translocation of the RNA-guided effector protein part of the complex through the pore results in an electrical effect or current effect that is one or more of (i) longer, (ii) more pronounced and (iii) more complex than the electrical effect or current effect associated with the target polymer part of the complex translocating through the pore.

4. A method according to claim 3, wherein a more complex electrical or current effect comprises one or more of (i) noise and (ii) electrical stepping events.

5. A method according to any one of claims 2-4, wherein the electrical or current effect associated with the target polymer is observed before and/or after the electrical or current effect associated with the RNA-guided effector protein.

6. A method according to any one of the preceding claims, wherein the target polymer is a target polynucleotide and wherein the target polynucleotide is optionally double stranded or comprises a double stranded region and/or is DNA, a DNA/RNA hybrid or RNA.

7. A method according to any one of the preceding claims, wherein the RNA-guided effector protein is an RNA-guided endonuclease or an RNA-guided endonuclease wherein the nuclease activity of the RNA-guided endonuclease is disabled and wherein optionally:
a) one or more catalytic nuclease sites of the RNA-guided endonuclease are inactivated; and/or
b) the RNA-guided endonuclease is Cas, Cas9, Cas12a or C2c2.

8. A method according to any one of the preceding claims, wherein the guide RNA comprises a nucleotide sequence that binds to a sequence in the target polymer and a nucleotide sequence that binds to the RNA-guided effector protein,
wherein the guide RNA comprises a crRNA that binds to a sequence in the target polynucleotide and a tracrRNA,
wherein the guide RNA is a sgRNA, or
wherein the guide RNA, the RNA-guided effector protein, or the target polymer, if present, has an anchor capable of coupling to the membrane, wherein the anchor optionally comprises cholesterol.

9. A method according to any one of the preceding claims, wherein step (a) comprises contacting the sample and the pore with two or more guide RNAs each of which (i) bind to a part of the target polymer and (ii) bind to or are attached to two or more RNA-guided effector proteins, wherein the two or more guide RNAs and two or more RNA-guided effector proteins form two or more complexes with the target polymer, if present, in the sample and step (d) comprises monitoring for the presence or absence of two or more electrical or current effects resulting from the translocation of the two or more complexes through the pore and thereby determining the presence or absence of the target polymer in the sample.

10. A method according to claim 9, wherein the two or more guide RNAs bind to different parts of the target polymer,
wherein the two or more RNA-guided effector proteins are different RNA-guided effector proteins, or
wherein the electrical or current effects associated with the target polymer part of the two or more complexes translocating through the pore are observed between the electrical or current effects resulting from the RNA-guided effector protein parts of the two or more complexes translocating through the pore.

11. A method according to any one of the preceding claims, further comprising determining the amount of the target polymer or one or more characteristics of the target polymer, if present, and wherein the one or more characteristics are optionally selected from (i) the length of the target polymer, (ii) the identity of the target polymer, (iii) the sequence of the target polymer, (iv) the secondary structure of the target polymer and (v) whether or not the target polymer is modified.

12. A method according to any one of the preceding claims, wherein the pore is a naturally occurring protein pore or a modified version of a naturally occurring protein pore.

13. A method according to claim 12, wherein the naturally occurring protein pore is Complement component 9 (C9), Perfringolysin O, Pleurotolysin, Listeriolysin, Perforin-2, Gasdermin-A3, L-, P- and M-ring protein, Type II secretion system protein D, GspD, InvG, VirB7, SpoIIIAG, Cag8, Cag3, Cag or other proteins in the Type IV secretion system apparatus protein CagY, WzzB, Pentraxin, Afp2, Major vault protein, Thioredoxin-dependent peroxidase reductase, Arf-GAP, Respiratory syncytial virus ribonucleoprotein, Chikungunya virus nonstructural protein 1, PRC, YaxA, XaxA or PrgH.

14. A method according to any one of the preceding claims, wherein the membrane is (i) a triblock copolymer membrane or (ii) a solid-state layer and the protein pore is present in a pore in the solid-state layer.

15. A system for determining the presence or absence of a target polymer in a sample comprising:
a) a guide RNA that (i) binds to a part of the target polymer, if present, and (ii) binds to or is attached to a RNA-guided effector protein, wherein the guide RNA and RNA-guided effector protein form a complex with the target polymer, if present, in the sample; and
b) a protein pore with an opening that permits translocation of the complex through the pore, wherein the pore is optionally present in a membrane.

## Patentansprüche

1. Verfahren zum Bestimmen des Vorhandenseins oder Nichtvorhandenseins eines Zielpolymers in einer Probe, umfassend:
a) Inkontaktbringen der Probe mit einer Leit-RNA, die (i) an einen Teil des Zielpolymers bindet und (ii) an ein RNA-geleitetes Effektorprotein bindet oder an dieses gebunden ist, wobei die Leit-RNA und das RNA-geleitete Effektorprotein einen Komplex mit dem Zielpolymer bilden, falls dieses in der Probe vorhanden ist;
b) Inkontaktbringen der Probe mit einer Membran, die eine Proteinpore mit einer Öffnung umfasst, die die Translokation des Komplexes durch die Pore ermöglicht;
c) Anlegen einer Potentialdifferenz über die Membran und Durchführen einer oder mehrerer elektrischer Messungen; und
d) Überwachen des Vorhandenseins oder Nichtvorhandenseins eines elektrischen Effekts, der aus der Translokation des Komplexes durch die Pore resultiert, und dadurch Bestimmen des Vorhandenseins oder Nichtvorhandenseins des Zielpolymers in der Probe.

2. Verfahren nach Anspruch 1, wobei Schritt (c) das Anlegen einer Potentialdifferenz über die Membran und das Messen des durch die Pore fließenden Stroms umfasst.

3. Verfahren nach Anspruch 1 oder 2, wobei die Translokation des RNA-geleiteten Effektorproteinteils des Komplexes durch die Pore zu einem elektrischen Effekt oder Stromeffekt führt, der (i) länger, (ii) ausgeprägter und (iii) komplexer ist als der elektrische Effekt oder Stromeffekt, der mit dem Zielpolymerteil des Komplexes verbunden ist, der durch die Pore transloziert.

4. Verfahren nach Anspruch 3, wobei ein komplexerer elektrischer oder Stromeffekt eines oder mehrere der folgenden Merkmale umfasst: (i) Rauschen und (ii) elektrische Sprungereignisse.

5. Verfahren nach einem der Ansprüche 2 bis 4, wobei der mit dem Zielpolymer verbundene elektrische oder Stromeffekt vor und/oder nach dem mit dem RNA-geleiteten Effektorprotein verbundenen elektrischen oder Stromeffekt beobachtet wird.

6. Verfahren nach einem der vorstehenden Ansprüche, wobei das Zielpolymer ein Zielpolynukleotid ist und wobei das Zielpolynukleotid wahlweise doppelsträngig ist oder einen doppelsträngigen Bereich umfasst und/oder DNA, ein DNA/RNA-Hybrid oder RNA ist.

7. Verfahren nach einem der vorstehenden Ansprüche, wobei das RNA-geleitete Effektorprotein eine RNA-geleitete Endonuklease ist oder eine RNA-geleitete Endonuklease, wobei die Nukleaseaktivität der RNA-geleiteten Endonuklease deaktiviert ist und wobei wahlweise:
a) eine oder mehrere katalytische Nuklease-Stellen der RNA-geleiteten Endonuklease inaktiviert sind; und/oder
b) die RNA-geleitete Endonuklease Cas, Cas9, Casl2a oder C2c2 ist.

8. Verfahren nach einem der vorstehenden Ansprüche, wobei die Leit-RNA eine Nukleotidsequenz umfasst, die an eine Sequenz im Zielpolymer bindet, und eine Nukleotidsequenz, die an das RNA-geleitete Effektorprotein bindet,
wobei die Leit-RNA eine crRNA umfasst, die an eine Sequenz im Zielpolynukleotid bindet, und eine tracrRNA, wobei die Leit-RNA eine sgRNA ist, oder
wobei die Leit-RNA, das RNA-geleitete Effektorprotein oder das Zielpolymer, falls vorhanden, einen Anker aufweist, der an die Membran koppeln kann, wobei der Anker wahlweise Cholesterin umfasst.

9. Verfahren nach einem der vorstehenden Ansprüche, wobei Schritt (a) das Inkontaktbringen der Probe und der Pore mit zwei oder mehr Leit-RNAs umfasst, von denen jede (i) an einen Teil des Zielpolymers bindet und (ii) an zwei oder mehr RNA-geleitete Effektorproteine bindet oder an diese gebunden ist, wobei die zwei oder mehr Leit-RNAs und die zwei oder mehr RNA-geleiteten Effektorproteine zwei oder mehr Komplexe mit dem Zielpolymer, falls vorhanden, in der Probe bilden, und Schritt (d) das Überwachen des Vorhandenseins oder Nichtvorhandenseins von zwei oder mehr elektrischen oder Stromeffekten umfasst, die aus der Translokation der zwei oder mehr Komplexe durch die Pore resultieren, und dadurch das Vorhandensein oder Nichtvorhandensein des Zielpolymers in der Probe bestimmt.

10. Verfahren nach Anspruch 9, wobei die zwei oder mehr Leit-RNAs an verschiedene Teile des Zielpolymers binden, wobei die zwei oder mehr RNA-geleiteten Effektorproteine verschiedene RNA-geleitete Effektorproteine sind, oder wobei die elektrischen oder Stromeffekte, die mit dem Zielpolymerteil der zwei oder mehr Komplexe verbunden sind, die durch die Pore translozieren, zwischen den elektrischen oder Stromeffekten beobachtet werden, die aus den RNA-geleiteten Effektorproteinteilen der zwei oder mehr Komplexe resultieren, die durch die Pore translozieren.

11. Verfahren nach einem der vorstehenden Ansprüche, das ferner das Bestimmen der Menge des Zielpolymers oder einer oder mehrerer Eigenschaften des Zielpolymers, falls vorhanden, umfasst, und wobei die eine oder mehreren Eigenschaften wahlweise ausgewählt sind aus (i) der Länge des Zielpolymers, (ii) der Identität des Zielpolymers, (iii) der Sequenz des Zielpolymers, (iv) der Sekundärstruktur des Zielpolymers und (v) der Frage, ob das Zielpolymer modifiziert ist oder nicht, ausgewählt werden.

12. Verfahren nach einem der vorstehenden Ansprüche, wobei die Pore eine natürlich vorkommende Proteinpore oder eine modifizierte Version einer natürlich vorkommenden Proteinpore ist.

13. Verfahren nach Anspruch 12, wobei die natürlich vorkommende Proteinpore Komplementkomponente 9 (C9), Perfringolysin O, Pleurotolysin, Listeriolysin, Perforin-2, Gasdermin-A3, L-, P- und M-Ring-Protein, Typ-II-Sekretionssystem-Protein D, GspD, InvG, VirB7, SpoIIIAG, Cag8, Cag3, Cag oder andere Proteine im Typ-IV-Sekretionssystem-Gerät-Protein CagY, WzzB, Pentraxin, Afp2, Major-Vault-Protein, Thioredoxin-unabhängige Peroxidase-Reduktase, Arf-GAP, Ribonukleoprotein des Respiratorischen Synzytial-Virus, Nichtstrukturprotein 1 des Chikungunya-Virus, PRC, YaxA, XaxA oder PrgH ist.

14. Verfahren nach einem der vorstehenden Ansprüche, wobei die Membran (i) eine Triblock-Copolymermembran oder (ii) eine Festphasenschicht ist und die Proteinpore in einer Pore in der Festphasenschicht vorhanden ist.

15. System zum Bestimmen des Vorhandenseins oder Nichtvorhandenseins eines Zielpolymers in einer Probe, umfassend:
a) eine Leit-RNA, die (i) an einen Teil des Zielpolymers bindet, falls vorhanden, und (ii) an ein RNA-geleitetes Effektorprotein bindet oder an dieses gebunden ist, wobei die Leit-RNA und das RNA-geleitete Effektorprotein einen Komplex mit dem Zielpolymer bilden, falls dieses in der Probe vorhanden ist; und
b) eine Proteinpore mit einer Öffnung, die die Translokation des Komplexes durch die Pore ermöglicht, wobei die Pore wahlweise in einer Membran vorhanden ist.

## Revendications

1. Procédé de détermination de la présence ou de l'absence d'un polymère cible dans un échantillon comprenant :
a) le contact de l'échantillon avec un ARN guide qui (i) se lie à une partie du polymère cible et (ii) se lie ou est fixé à une protéine effectrice guidée par ARN, dans lequel l'ARN guide et la protéine effectrice guidée par ARN forment un complexe avec le polymère cible, s'il est présent, dans l'échantillon ;
b) le contact de l'échantillon avec une membrane comprenant un pore protéique avec une ouverture qui permet la translocation du complexe à travers le pore ;
c) l'application d'une différence de potentiel à travers la membrane et la prise d'une ou plusieurs mesures électriques ; et
d) la surveillance de la présence ou de l'absence d'un effet électrique résultant de la translocation du complexe à travers le pore et ainsi la détermination de la présence ou de l'absence du polymère cible dans l'échantillon.

2. Procédé selon la revendication 1, dans lequel l'étape (c) comprend l'application d'une différence de potentiel à travers la membrane et la mesure du courant traversant le pore.

3. Procédé selon la revendication 1 ou 2, dans lequel la translocation de la partie protéique effectrice guidée par ARN du complexe à travers le pore entraîne un effet électrique ou un effet de courant qui est l'un des éléments suivants : (i) plus long, (ii) plus prononcé et (iii) plus complexe que l'effet électrique ou l'effet de courant associé à la partie polymère cible du complexe subissant une translocation à travers le pore.

4. Procédé selon la revendication 3, dans lequel un effet électrique ou de courant plus complexe comprend l'un ou plusieurs des évènements (i) de bruit et (ii) de pas électriques.

5. Procédé selon l'une quelconque des revendications 2 à 4, dans lequel l'effet électrique ou de courant associé au polymère cible est observé avant et/ou après l'effet électrique ou de courant associé à la protéine effectrice guidée par ARN.

6. Procédé selon l'une quelconque des revendications précédentes, dans lequel le polymère cible est un polynucléotide cible et dans lequel le polynucléotide cible est éventuellement double brin ou comprend une région double brin et/ou est de l'ADN, un hybride ADN/ARN ou de l'ARN.

7. Procédé selon l'une quelconque des revendications précédentes, dans lequel la protéine effectrice guidée par ARN est une endonucléase guidée par ARN ou une endonucléase guidée par ARN dans lequel l'activité nucléasique de l'endonucléase guidée par ARN est désactivée et dans lequel éventuellement :
a) un ou plusieurs sites de nucléase catalytiques de l'endonucléase guidée par ARN sont inactivés ; et/ou
b) l'endonucléase guidée par ARN est Cas, Cas9, Casl2a ou C2c2.

8. Procédé selon l'une quelconque des revendications précédentes, dans lequel l'ARN guide comprend une séquence nucléotidique qui se lie une séquence du polymère cible et une séquence nucléotidique qui se lie à la protéine effectrice guidée par ARN,
dans lequel l'ARN guide comprend un crARN qui se lie à une séquence du polynucléotide cible et un tracrARN, dans lequel l'ARN guide est un sgARN, ou
dans lequel l'ARN guide, la protéine effectrice guidée par ARN, ou le polymère cible, s'il est présent, a un ancrage capable de se coupler à la membrane, dans lequel l'ancrage comprend éventuellement du cholestérol.

9. Procédé selon l'une quelconque des revendications précédentes, dans lequel l'étape (a) comprend la mise en contact de l'échantillon et du pore avec deux ARN guides ou plus chacun d'eux (i) se liant à une partie du polymère cible et (ii) se liant à ou étant fixé à deux protéines effectrices guidées par ARN ou plus, dans lequel les deux ARN guides ou plus et les deux protéines effectrices guidées par ARN ou plus forment deux complexes ou plus avec le polymère cible, s'il est présent, dans l'échantillon et l'étape (d) comprend la surveillance de la présence ou de l'absence de deux effets électriques ou de courant ou plus résultant de la translocation des deux complexes ou plus à travers le pore et ainsi la détermination de la présence ou de l'absence du polymère cible dans l'échantillon.

10. Procédé selon la revendication 9, dans lequel les deux ARN guides ou plus se lient à différentes parties du polymère cible,
dans lequel les deux protéines effectrices guidées par ARN ou plus sont des protéines effectrices guidées par ARN différentes, ou
dans lequel les effets électriques ou de courant associés à la partie polymère cible des deux complexes ou plus subissant une translocation à travers le pore sont observés entre les effets électriques ou de courant résultant des parties protéiques effectrices guidées par ARN des deux complexes ou plus subissant une translocation à travers le pore.

11. Procédé selon l'une quelconque des revendications précédentes, comprenant en outre la détermination de la quantité du polymère cible ou d'une ou plusieurs caractéristiques du polymère cible, s'il est présent, et dans lequel les une ou plusieurs caractéristiques sont éventuellement choisies parmi (i) la longueur du polymère cible, (ii) l'identité du polymère cible, (iii) la séquence du polymère cible, (iv) la structure secondaire du polymère cible et (v) si le polymère cible est modifié ou non.

12. Procédé selon l'une quelconque des revendications précédentes, dans lequel le pore est un pore protéique naturel ou une version modifiée d'un pore protéique naturel.

13. Procédé selon la revendication 12, dans lequel le pore protéique naturel est le composant de complément 9 (C9), la perfringolysine O, la pleurotolysine, la listériolysine, la perforine-2, la gasdermine-A3, la protéine annulaire L, P et M, la protéine D du système de sécrétion de type II, GspD, InvG, VirB7, SpoIIIAG, Cag8, Cag3, Cag ou d'autres protéines de l'appareil du système de sécrétion de type IV, la protéine CagY, WzzB, la pentraxine, Afp2, la protéine de voûte majeure, la peroxydase réductase indépendante de la thioredoxine, Arf-GAP, la ribonucléoprotéine du virus respiratoire syncytial, la protéine non structurale 1 du virus Chikungunya, PRC, YaxA, XaxA ou PrgH.

14. Procédé selon l'une quelconque des revendications précédentes, dans lequel la membrane est (i) une membrane de copolymère tribloc ou (ii) une couche à l'état solide et le pore protéique est présent dans un pore de la couche à l'état solide.

15. Système permettant de déterminer la présence ou l'absence d'un polymère cible dans un échantillon comprenant :
a) un ARN guide qui (i) se lie à une partie du polymère cible, s'il est présent, et (ii) se lie ou est fixé à une protéine effectrice guidée par ARN, dans lequel l'ARN guide et la protéine effectrice guidée par ARN forment un complexe avec le polymère cible, s'il est présent, dans l'échantillon ; et
b) un pore protéique avec une ouverture qui permet la translocation du complexe à travers le pore, dans lequel le pore est éventuellement présent dans une membrane.
